(19) Europäisches Patentamt
European Patent Office
Office européen des brevets

(11) **EP 3 167 918 B1**

(12) **EUROPEAN PATENT SPECIFICATION**

(45) Date of publication and mention
of the grant of the patent:
**06.06.2018 Bulletin 2018/23**

(21) Application number: **15819761.6**

(22) Date of filing: **06.07.2015**

(51) Int Cl.:
*A61M 1/02* (2006.01)   *A61M 1/34* (2006.01)
*A61M 1/36* (2006.01)

(86) International application number:
**PCT/JP2015/069417**

(87) International publication number:
**WO 2016/006574 (14.01.2016 Gazette 2016/02)**

(54) **BLOOD PROCESSING FILTER AND METHOD FOR PRODUCING BLOOD PROCESSING FILTER**

BLUTAUFBEREITUNGSFILTER UND VERFAHREN ZUR HERSTELLUNG DES
BLUTAUFBEREITUNGSFILTERS

FILTRE DE TRAITEMENT DE SANG ET PROCÉDÉ DE PRODUCTION DE FILTRE DE TRAITEMENT
DE SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.07.2014   JP 2014139885**
**07.07.2014   JP 2014139886**

(43) Date of publication of application:
**17.05.2017   Bulletin 2017/20**

(73) Proprietor: **Asahi Kasei Medical Co., Ltd.
Chiyoda-ku
Tokyo 101-8101 (JP)**

(72) Inventor: **MATSUURA, Yoshimasa
Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
**WO-A1-2011/122564      WO-A1-2015/050213
WO-A1-2015/050215      JP-A- S52 116 969
JP-A- 2001 226 271      JP-A- 2001 226 271
JP-A- 2003 019 732      JP-A- 2007 097 746
JP-A- 2011 072 814      JP-A- 2011 072 816
JP-A- 2011 255 043      JP-A- 2012 139 347
US-A- 4 157 967      US-B1- 6 168 718**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Technical Field**

The present invention relates to a blood processing filter for removing undesirable components, such as aggregates and leukocytes, from liquid containing blood components or blood, and to a blood processing filter manufacturing method. In particular, the blood processing filter is, for example, a disposable blood processing filter, and is used for the sake of removing microaggregates and leukocytes which may cause side effects from whole blood preparations, erythrocyte preparations, thrombocyte preparations, blood plasma preparations and the like for blood transfusion.

**Background Art**

It is becoming common for whole blood collected from a donor to be separated into blood component preparations, such as an erythrocyte preparation, a thrombocyte preparation, and a blood plasma preparation, stored and then provided for transfusion. Since microaggregates and leukocytes included in these blood preparations cause various side effects of blood transfusion, many methods of removing these undesirable components before transfusion and then performing transfusion, or of removing these undesirable components after blood collection, temporarily storing the preparations, and then providing the preparations for transfusion have been widely used.

Among methods of removing these undesirable components from blood preparations, processing blood preparations through a blood processing filter is most typical. Two types of blood processing filters are used; one is what includes a filter element made of nonwoven fabric or a porous body equipped in, for example, a flexible container as described in Patent Literatures 1 to 5, and the other is what includes the filter element equipped in a hard container made of polycarbonate or the like.

Typically, for processing of a blood preparation through a blood processing filter, a blood preparation bag containing the blood preparation to be processed is connected to an inlet of the blood processing filter, the blood preparation bag is placed at a position higher than the blood processing filter by approximately 20 to 100 cm. As a result, the blood preparation is introduced from the blood preparation bag into the blood processing filter by the action of gravity. Meanwhile, a recovery bag for accommodating the filtered blood preparation is connected to an outlet of the blood processing filter. The recovery bag is placed at a lower position by approximately 50 to 100 cm than that of the blood processing filter. As a result, the filtered blood preparation is collected in the recovery bag by the action of gravity. At this time, a pressure loss occurs due to the resistance of the filter element, in an inlet-side space in the blood processing filter container with respect to the filter element, whereby the space becomes a positive pressure. On the contrary, in an outlet-side space with respect to the filter element, the blood preparation flows from the outlet, whereby the space becomes a negative pressure.

Since a blood processing filter with a flexible container as shown in Patent Literatures 1 to 5 has a container that is flexible, the container swells like a balloon due to a positive pressure in an inlet-side space, and the filter element is pressed against on the outlet side of the container. On the other hand, in an outlet-side space, the container is in close contact with the filter element due to a negative pressure, and the state is brought into that where the opening of the outlet is blocked. That is, since blood tends to flow from the filter but the opening is blocked, it is difficult for the blood to flow out.

On the contrary, a blood processing filter with a hard container does not largely deform even during filtration, the filter element is not pressed against the outlet-side, and the state is not brought into that where the opening of the outlet is blocked. Such a hard container includes an inlet-side container element and an outlet-side container element which are fitted with each other, and rib-shaped convexes provided for the inlet-side container element and the outlet-side container element are pressed against each other, thereby clamping the outer edge portion of the filter element. By pressing the convexes against each other to a high density, the side leakage (side flow) where blood flows over the outer edge portion of the filter element without being filtered can be prevented.

Incidentally, the entire apparatus is required to be sterilized in order to be used for processing blood preparations. Typically, steam sterilization is applied. The filter for the hard container has a poor steam permeability, and is required to be subjected to a long sterilization time. However, execution of a long-time autoclave sterilization causes degradation of blood stock solution and the like. Accordingly, after the filter is sterilized in a separated manner and the blood bag, circuit and the like are then connected, sterilization is required to be performed again. Thus, complicated operations are required to be executed.

A filter described in Patent Literature 6 has been invented as means for solving this. In this filter, a part of or the entire inlet-side container element is made of a flexible member, and a part of or the entire outlet-side container element is made of a hard member. In this filter, adoption of the hard member for the outlet-side container element can prevent a poor blood flow due to close contact of the outlet-side container element with the filter element, while adoption of the flexible member for the inlet-side container element can secure steam permeability during steam sterilization.

**Citation List**

**Patent Literature**

[Patent Literature 1] Japanese Unexamined Patent Publication No. H11-216179
[Patent Literature 2] Japanese Unexamined Patent Publication No. H7-267871
[Patent Literature 3] International Publication No. WO 2004/050147
[Patent Literature 4] International Publication No. WO 95/17236
[Patent Literature 5] European Unexamined Patent Publication No. 0526678
[Patent Literature 6] Japanese Unexamined Patent Publication No. 2011-072814

**Summary of Invention**

**Technical Problem**

However, as a result of verification by the present inventors, it has become apparent that there is a possibility that in the filter that concurrently adopts a flexible member and a hard member for parts of or the entire inlet-side container element and the outlet-side container element, contraction of resin being subject to steam at high temperature and high pressure affects the adhering portions of the inlet-side container element, the filter element, and the outlet-side container element, reduces the performance of removing undesirable components, and breakage of the container occurs.

Thus, a first and a third aspect of the present invention have an object to provide a blood processing filter that can easily avoid the risk of incomplete removal of undesirable components without reducing the blood processing efficiency and is suitable for autoclave sterilization, and to provide a blood processing filter manufacturing method.

In the above background art, an ultrasonic bonding method is mainly used for adhesion of the hard container. However, in view of energy transmission efficiency, as only the peripheral portion is pressed and bonded. Deformation of the container easily occurs, and there is a tendency where, in both the filter and the filter element after molding, the central portion is thick and the peripheral side is thin, and the blood flow during blood processing tends to be uneven.

Thus, a second and a fourth aspect of the present invention have an object to provide a blood processing filter that can uniformly use the filter element and is suitable for autoclave sterilization, and a blood processing filter manufacturing method.

**Solution to Problem**

As a result of diligent research by the present inventors to solve the problems, they have found that the risk of incomplete removal of undesirable components can be easily avoided without reducing the blood processing efficiency by fabricating the main parts of the inlet-side container element and the outlet-side container element to be made of a hard resin and by causing the inlet-side container element and the outlet-side container element to clamp the outer edge portions of the front and back of filtering spaces of the filter element to allow the portions to have a predetermined thickness. Furthermore, the inventors have found knowledge that the autoclave sterilization can be improved by providing a steam-permeable portion that has steam permeability for a part of the hard container formed of the inlet-side container element and the outlet-side container element, and thus reached an aspect of the present invention.

That is, a first aspect of the present invention is a blood processing filter for removing undesirable components from liquid containing a blood component or blood, the filter comprising: a sheet-shaped filter element; and a hard container that includes an inlet-side container element and an outlet-side container element that are disposed to clamp the filter element, and has an internal space separated by the filter element into an inlet space and an outlet space, wherein the filter element includes a pair of filtering surfaces disposed on the inlet space side and the outlet space side, respectively, the inlet-side container element and the outlet-side container element are provided with a gripper that clamps and compresses outer edge portions of the pair of filtering surfaces, the filter element is compressed to have a thickness of a portion clamped by the gripper being 0.05 times or more and 0.5 times or less larger, and the hard container is provided with a steam-permeable portion having steam permeability. Note that, blood according to the present invention includes blood preparations, such as whole blood preparations, erythrocyte preparations, thrombocyte preparations and blood plasma preparations, for blood transfusion.

The hard container of the blood processing filter according to the first aspect is provided with the steam-permeable portion having steam permeability. Consequently, sterilization in an autoclave can be easily executed. The inlet-side container element and the outlet-side container element are provided with the gripper which clamps and compresses the outer edge portions of the pair of filtering surfaces. The filter element is compressed by the gripper to have a thickness 0.05 to 0.5 times larger. As a result, the side leakage (side flow) where undesirable components flow over the outer edge portions of the filter element without being filtered can be prevented.

The first aspect may be the blood processing filter wherein the steam-permeable portion is a film that has steam permeability and is provided for at least one of the inlet-side container element and the outlet-side container element.

The first aspect may be the blood processing filter wherein the inlet-side container element and the outlet-side container element are made of a hard resin, and the steam-permeable portion is made of a flexible resin.

The first aspect may be the blood processing filter wherein the steam-permeable portion is a part of the hard container and is a thin-walled portion that is thinner than other portions, and the steam-permeable portion and the other portions are integrally formed.

Furthermore, in the first aspect may be the blood processing filter wherein the steam-permeable portion has a thickness of 50 $\mu$m or more and 500 $\mu$m or less.

The first aspect may be the blood processing filter wherein a coefficient of variation value (CV value) of a thickness of the filter element is 6% or less. Consequently, the filter element can be effectively utilized. In the case where the difference in thickness of the filter element is large during filtering blood, the resistance of a thick portion, i.e., a portion with a low bulk density is low, and the blood passes only through the thick portion of the filter element. Consequently, an imbalanced flow can be prevented, and the risk that the entire filter cannot be used can be easily avoided.

The first aspect may be the blood processing filter wherein a coefficient of variation value (CV value) of a distance from the inlet-side container element to an inlet-side surface of the filter element in the inlet space is 10% or less. Consequently, blood entering from the inlet uniformly spreads into the inlet space, and the filter element can be effectively used.

A second aspect of the present invention is a blood processing filter for removing undesirable components from liquid containing a blood component or blood, the filter comprising: a sheet-shaped filter element; and a hard container that includes an inlet-side container element and an outlet-side container element that are disposed to clamp the filter element, and has an internal space separated by the filter element into an inlet space and an outlet space, wherein the filter element includes a filtering surface on a side of the inlet space, a filtering surface on a side of the outlet space, and an end surface along peripheries of the pair of filtering surfaces, the inlet-side container element and the outlet-side container element are provided with a gripper that clamps and compresses outer edge portions of the pair of filtering surfaces, a coefficient of variation value of the thickness of the filter element is 6% or less, and the hard container is provided with a steam-permeable portion having steam permeability.

In the case where the difference in thickness among positions of the filter element is large during filtering blood and the like, the resistance of a thick portion, i.e., a portion with a low bulk density is low, and the blood and the like pass only through thick portions of the filter element. Consequently, a drifting flow easily occurs, which causes a risk that the filter cannot be entirely used. Unlike this, since the aforementioned producing method sets the coefficient of variation value (CV value) of the thickness of the filter element to 6% or less, the blood processing filter according to the second aspect can prevent the drifting flow of blood and the like, and effectively utilize the entire filter element. Furthermore, the hard container of the blood processing filter according to the second aspect is provided with the steam-permeable portion having steam permeability. Consequently, sterilization in an autoclave can be easily executed.

The second aspect may be the blood processing filter wherein a coefficient of variation value (CV value) of a distance from the inlet-side container element to an inlet-side surface of the filter element in the inlet space is 10% or less. Consequently, blood entering from the inlet uniformly spreads into the inlet space, and the filter element can be effectively used.

The second aspect may be the blood processing filter wherein the inlet-side container element and the outlet-side container element are made of a hard resin, and the steam-permeable portion is made of a flexible resin.

The second aspect may be the blood processing filter wherein the steam-permeable portion is a part of the hard container and is a thin-walled portion that is thinner than other portions, and the steam-permeable portion and the other portions are integrally formed.

The second aspect may be the blood processing filter wherein the steam-permeable portion has a thickness of 50 $\mu$m or more and 500 $\mu$m or less.

The second aspect may be the blood processing filter wherein the filter element includes an end surface along a periphery of the pair of filtering surfaces, and the gripper includes a close contact portion that is in close contact with the end surface. The close contact portion that is in close contact with the end surface is thus provided. Consequently, the gripper is positioned and is resistant to deviate, which is advantageous in high-density compression of the outer edge portions of the pair of filtering surfaces against each other. As a result, even if the region of the outer edge portions compressed by the gripper is configured to be small, compression can be securely performed by the gripper. Consequently, the risk of incomplete removal of undesirable components can be easily avoided without reducing the blood processing efficiency.

The second aspect may be the blood processing filter wherein the gripper compresses the filter element at a range with a width of 1 to 5 mm from an outermost edge of the filter element, as the outer edge portion. The range of the outer edge portion is defined as the aforementioned range. Consequently, the risk of incomplete removal of undesirable components can be easily avoided without reducing the blood processing efficiency.

The second aspect may be the blood processing filter wherein the inlet-side container element and the outlet-side container element further include a fitting portion that surrounds the end surface of the filter element to be fitted thereto. The fitting portions are thus fitted to each other. Consequently, the inlet-side container element and the outlet-side container element can be easily aligned with each other. Furthermore, the inlet-side container element and the outlet-side container element can be easily joined.

The second aspect may be the blood processing filter wherein the fitting portion includes an inlet-side fitting portion provided at the inlet-side container element, and an outlet-side fitting portion provided at the outlet-side container element, and at least a part of a sliding contact portion between the inlet-side fitting portion and the outlet-side fitting portion is bonded in a belt shaped manner along an entire periphery of the fitting portion with melt resin. The blood processing filter has improved airtightness and liquid-tightness, and is preferable.

The second aspect may be the blood processing filter wherein the sliding contact portion is provided with a resin flow path, the resin flow path is filled with the melt resin to bond the sliding contact portion. In this blood processing filter, the melt resin is resistant to deviating from the sliding contact portion, has no possibility of entering the inside of the hard container, and the filter is preferable.

The second aspect may be the blood processing filter wherein the hard container is made of a resin with a tensile elastic modulus of at least 1 GPa at room temperature. The blood processing filter can reduce the deformation during filtration, and is preferable.

Furthermore, a third aspect of the present invention is a blood processing filter manufacturing method, the filter being for removing undesirable components from liquid containing a blood component or blood. This blood processing filter comprises a sheet-shaped filter element and a hard container that includes an inlet-side container element and an outlet-side container element that are disposed to clamp the filter element, and has an internal space separated by the filter element into an inlet space and an outlet space, wherein the filter element includes a pair of filtering surfaces disposed on the inlet space side and the outlet space side, the inlet-side container element and the outlet-side container element are provided with a gripper that clamps and compresses outer edge portions of the pair of filtering surfaces, the filter element is compressed by the gripper to have a thickness of being 0.05 to 0.5 times larger, and the hard container is provided with a steam-permeable portion having steam permeability. The blood processing filter manufacturing method according to the third embodiment comprises: a container element molding step of injection-molding the inlet-side container element with one mold and injection-molding the outlet-side container element with another mold, and forming a steam-permeable portion in at least one of the inlet-side container element and the outlet-side container element; a inserting step of inserting a filter element into the inlet-side container element or the outlet-side container element; a joining step of bringing the inlet-side container element and the outlet-side container element into contact with each other in a state where the filter element is loaded, and compressing an outer edge portion of the filter element to have a thickness of being 0.05 to 0.5 times larger; and a fixing step of fixing the inlet-side container element and the outlet-side container element to each other.

The blood processing filter manufacturing method according to a third aspect comprises the joining step and the adhesion step. Accordingly, the inlet-side container element and the outlet-side container element can be pressed against each other with a strong power. Consequently, the filter element can be compressed to have a high density. Furthermore, in the state of being compressed to have the high density, the inlet-side container element, the outlet-side container element and the end surface of the filter element can be caused to adhere to each other with melt resin. As a result, the blood processing filter can effectively prevent a side leakage (side flow) where undesirable components flow over the outer edge portion of the filter element without being filtered. Furthermore, at least one of the inlet-side container element and the outlet-side container element is thus provided with the steam-permeable portion. Consequently, sterilization in an autoclave can be easily executed.

The third aspect may be the blood processing filter wherein the container element molding step forms the steam-permeable portion by providing a film having steam permeability for at least one of the inlet-side container element and the outlet-side container element.

The third aspect may be the blood processing filter wherein the container element molding step forms the steam-permeable portion by providing a thin-walled portion that is a part thinner than other portions for at least one of the inlet-side container element and the outlet-side container element.

The third aspect may be the blood processing filter wherein the joining step includes a fitting step of mold-fitting the set of molds to fit the inlet-side container element and the outlet-side container element to each other in a state of clamping the filter element. The fitting step of mold-closing the set of molds and fitting the inlet-side container element and the outlet-side container element to each other is thus included. Accordingly, the inlet-side container element and the outlet-side container element can be pressed against each other with a strong power. Consequently, the filter element can be compressed to have a higher density.

Furthermore, a fourth aspect of the present invention is a blood processing filter manufacturing method, the filter being for removing undesirable components from liquid containing a blood component or blood. This blood processing filter comprises a sheet-shaped filter element and a hard container that includes an inlet-side container element and an outlet-side container element that are disposed to clamp the filter element, and has an internal space separated by the filter element into an inlet space and an outlet space, wherein the filter element includes a pair of filtering surfaces disposed on the inlet space side and the outlet space side, the inlet-side container element and the outlet-side container element are provided with a gripper that clamps and compresses outer edge portions of the pair of filtering surfaces, a coefficient of variation value of a thickness of the filter element is 6% or less, and the hard container is provided with a steam-permeable portion having steam permeability. The blood processing filter manufacturing method according to the fourth aspect comprises: a container element molding step of injection-molding the inlet-side container element with one mold and injection-molding the outlet-side container element with another mold, and forming a steam-permeable portion in at least one of the inlet-side container element and the outlet-side container element; a inserting step of inserting a filter element into the inlet-side container element or the outlet-side container element; a joining step of bringing the inlet-side container element and the outlet-side container element into contact with each other in a state where the filter element is loaded, and compressing an outer edge portion of the filter element to have a coefficient of variation value being 6% or less; and a fixing step of fixing the inlet-side container element and the outlet-side container element to each other.

According to the blood processing filter manufacturing method of the fourth aspect, the fitting step of closing the set of molds and fitting the inlet-side container element and the outlet-side container element, thereby allowing the inlet-side container element and the outlet-side container element to be pressed against each other. Consequently, the filter element can be compressed to have a high density. Furthermore, the side leakage (side flow) where undesirable components flow over the outer edge portion of the filter element without being filtered can be prevented by the gripper of the filter element. Moreover, in the case where the difference in thickness of the filter element is large during filtering blood, the resistance of a thick portion, i.e., a portion with a low bulk density is low, and the blood pass only through the thick portion of the filter element. Consequently, an imbalanced flow can be prevented, and the risk that the filter cannot be entirely used can be easily avoided. Furthermore, at least one of the inlet-side container element and the outlet-side container element is thus provided with the steam-permeable portion. Consequently, sterilization in an autoclave can be easily executed.

The fourth aspect may be the blood processing filter wherein the container element molding step forms the steam-permeable portion by providing a film having steam permeability for at least one of the inlet-side container element and the outlet-side container element.

The fourth aspect may be the blood processing filter wherein the container element molding step forms the steam-permeable portion by providing a thin-walled portion that is a part thinner than other portions for at least one of the inlet-side container element and the outlet-side container element.

The fourth aspect may be the blood processing filter wherein the joining step includes a fitting step of mold-fitting the set of molds to fit the inlet-side container element and the outlet-side container element to each other in a state of clamping the filter element. The fitting step of mold-closing the set of molds and fitting the inlet-side container element and the outlet-side container element to each other is thus included. Accordingly, the inlet-side container element and the outlet-side container element can be pressed against each other with a strong power. Consequently, the filter element can be compressed to have a higher density.

### Advantageous Effects of Invention

The first and third aspects of the present invention can easily avoid the risk of incomplete removal of undesirable components without reducing the blood processing efficiency, and improve autoclave sterilization. The second and fourth aspects of the present invention can uniformly use the filter element, and further improve autoclave sterilization.

### Brief Description of Drawings

[Figure 1] Figure 1 is a plan view of a blood processing filter in view from the inlet-side container element according to an embodiment of the present invention.
[Figure 2] Figure 2 is a side view of the blood processing filter according to the embodiment of the present invention.
[Figure 3] Figure 3 is a sectional view taken along line III-III of Figure 1 and shows steam-permeable portions and a fitting portion in an enlarged manner.
[Figure 4] Figure 4 corresponds to Figure 3 and is a sectional view showing a first variational example of a steam-permeable portion according to this embodiment in an enlarged manner.
[Figure 5] Figure 5 corresponds to Figure 3 and is a sectional view showing a variational example of a fitting portion according to this embodiment in an enlarged manner.

[Figure 6] Figure 6 shows a measurement result of measurement on the thickness of a filter element before and after molding.

[Figure 7] Figure 7 is an illustration diagram for illustrating a container element molding step in manufacturing the blood processing filter according to the embodiment.

[Figure 8] Figure 8 is an illustration diagram for illustrating a inserting step in manufacturing the blood processing filter according to the embodiment.

[Figure 9] Figure 9 is an illustration diagram for illustrating a fitting step (joining step) and a fixing step in manufacturing the blood processing filter according to the embodiment of the present invention.

[Figure 10] Figure 10 is a sectional view of the blood processing filter for illustrating the coefficient of variation value (CV value) of the thickness of the filter element and the coefficient of variation value (CV value) of the distance from an inlet-side container element to an inlet-side surface of the filter element.

[Figure 11] Figure 11 corresponds to Figure 3 and is a sectional view showing a second variational example of a steam-permeable portion according to this embodiment in an enlarged manner.

[Figure 12] Figure 12 is corresponds to Figure 3 and is a sectional view of a blood processing filter that includes the steam-permeable portion according to the second variational example and is provided with a fitting portion according to a variational example.

## Description of Embodiments

Hereinafter, referring to the drawings, preferred embodiments of a blood processing filter and a blood processing filter manufacturing method according to the present invention are described in detail.

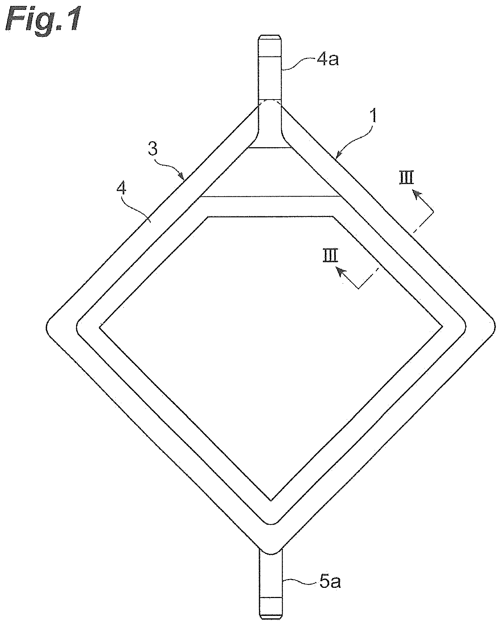

First, a blood processing filter 1 according to an embodiment of the present invention is described referring to Figures 1 to 3. The blood processing filter 1 is for removing undesirable components from liquid containing blood components or blood (hereinafter, referred to as liquid to be processed).

The blood processing filter 1 has a rectangular shape as a whole, and includes a sheet-shaped filter element 2 and a hard container 3. The hard container 3 includes an inlet-side container element 4 and an outlet-side container element 5 that are disposed to clamp the filter element 2 and are fixed to each other. An internal space 3s is separated by the filter element 2 into an inlet space 4s and an outlet space 5s. The inlet-side container element 4 is provided with an inlet port 4a that introduces the liquid to be processed into the inside. The outlet-side container element 5 is provided with an outlet port 5a that discharges the liquid processed through the filter element 2. Fixing means a concept including bonding and adhesion. Although the blood processing filter 1 may have any of a rectangular shape, a disk shape, an elliptic shape and the like, the rectangular shape is preferable to reduce the loss of material during production. A square shape and a rhombus are regarded as types of rectangular shapes.

In order to prevent deformation during filtration, it is preferable that the materials of the inlet-side container element 4 and the outlet-side container element 5 be a resin having a tensile elastic modulus of at least 1 GPa at room temperature, and it is further preferable that the materials be a resin having a tensile elastic modulus of at least 2 GPa. For example, polycarbonate, polyester, polyamide, polystyrene, HIPS, ABS, polyethylene, polypropylene, polyvinyl chloride or the like may be used. Heat-resistant polycarbonate or the like is further preferable. As to measurement of tensile elastic modulus, a result of measurement according to ISO527-1 can be used.

(About steam-permeable portion of hard container)

At the hard container 3, steam-permeable portions 31 having steam permeability (characteristics of allowing steam to permeate) are formed. The formation of the steam-permeable portions 31 allows steam to enter the hard container 3 (inside of the blood processing filter 1) during execution of autoclave sterilization, and enables easy sterilization in the blood processing filter 1. An example of the steam-permeable portion 31 is described in detail.

Openings 32 are formed at about the centers of the inlet-side container element 4 and the outlet-side container element 5. The inlet-side container element 4 and the outlet-side container element 5 are each provided with a film 31a having steam permeability to block the openings 32. The film 31a having steam permeability (characteristics of allowing steam to permeate) is arranged at the inlet-side container element 4 or the outlet-side container element 5 to form the steam-permeable portion 31, which allows steam to enter the hard container 3 (inside of the blood processing filter 1) during execution of autoclave sterilization, and enables easy sterilization in the blood processing filter 1. The steam-permeable portion 31 may be formed of a flexible resin having steam permeability. In this embodiment, this portion is formed using the film 31a made of a flexible resin, for example. In this embodiment, the mode has been described where the steam-permeable portion 31 is formed at both the inlet-side container element 4 and the outlet-side container element 5. Alternatively, a mode may be adopted where the steam-permeable portion 31 is formed at one of the inlet-side container element 4 and the outlet-side container element 5.

Examples of the material of the film 31a having steam permeability include polyvinyl chloride, hydrogenated styrene thermoplastic elastomer, polyolefin such as polyethylene and polypropylene, polyester such as polyethylene terephthalate, and resin such as polycarbonate, or the film may be a film 31a or the like usable for high pressure steam sterilization, such as Tyvek (R) made by pressing high-density polyethylene nonwoven fabric to achieve liquid-impermeability.

The gas permeable coefficient = the amount of gas permeation (volume) × thickness of steam-permeable portion (e.g., film 31a)/(pressure difference × permeation area × time). Accordingly, reduction in thickness of the steam-permeable portion 31 and increase in area can improve the steam permeability. Consequently, it is important to design the amount of gas (steam) permeation to be at least a certain amount (e.g., at least 0.50 g (measurement conditions: room temperature, 1 atm, and a day)) to achieve the thickness and area of the steam-permeable portion 31 that correspond to time during which autoclave sterilization is executed. An internal pressure occurs during filtration. Consequently, the strength for enduring the pressure is also required to be considered.

In this embodiment, ribs 4h and 5h are provided between the respective films 31a having steam permeability and the filter element 2. As a result, even if the internal pressure in the blood processing filter 1 becomes a negative pressure during filtration, the film 31a that allows steam to permeate is not pressed against the filter element 2. Consequently, it becomes unlikely to cause extension of filtering time and the like.

According to the method of forming the steam-permeable portion 31 at a part of any or both of the inlet-side container element 4 and the outlet-side container element 5 by providing the films 31a, the film 31a having steam permeability may be insertion-molded during molding of the container element through injection molding. Parts of the inlet-side container element 4 and the outlet-side container element 5 are hollowed. The film 31a may be caused to adhere thereon through a bonding method, such as of adhesives, high frequency or ultrasonic waves. In this case, insertion-molding is better because the insertion-molding can reduce steps. In the case where the materials of the inlet-side container element 4 and the outlet-side container element 5 are materials that tend to allow steam to permeate, the steam-permeable portion 31 can be formed even without use of the film 31a. For example, in some cases, it is only required to form thin-walled portions to further improve the steam permeabilities of the inlet-side container element 4 and the outlet-side container element 5 themselves. In such cases, the thickness of the thin-walled portion may be a thickness ranging from 50 to 500 μm, inclusive, to improve the steam permeability.

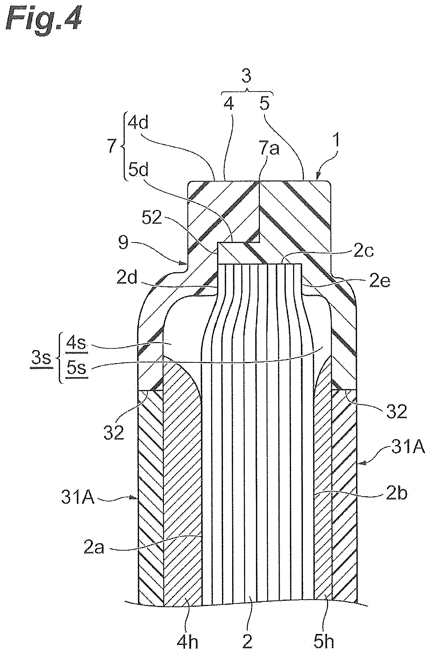

As in a first variational example of the steam-permeable portion 31, for example, as shown in Figure 4, an opening 32 for autoclave sterilization may be formed at a part of one of or parts of both the inlet-side container element 4 and the outlet-side container element 5 which are formed of a hard resin, a flexible resin having steam permeability may be provided to block the opening 32, thereby allowing a steam-permeable portion 31A to be formed.

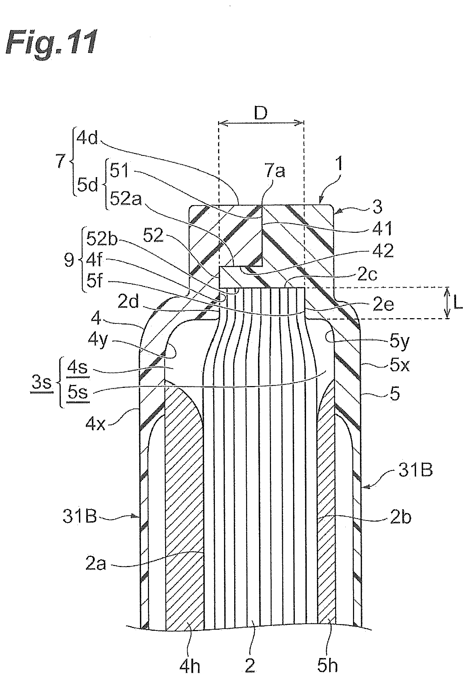

As a second variational example of the steam-permeable portion 31, for example, as shown in Figure 11, parts 31B of the inlet-side container element 4 and the outlet-side container element 5 are thin-walled portions that have smaller thickness (wall thickness) than another portion 31X. The thin-walled portion may be integrally formed with the other portion (thick wall portion) 31X. The thickness of the thin-walled portion is configured to be small to an extent of having steam permeability (characteristics of allowing steam to permeate). As a result, the thin-walled portion serves as the steam-permeable portion 31B.

More specifically, although there is some variation according to the materials of the inlet-side container element 4 and the outlet-side container element 5, it is preferable that the thickness of the steam-permeable portion 31B serving as the thin-walled portion range from 50 to 500 μm, inclusive. This is because in the case of 50 μm or less, the resin does not flow in during molding and difficulty in molding occurs, and in the case of exceeding 500 μm, the effect of steam permeability is reduced. The reduction in steam permeability means reduction to an extent of insufficient sterilization results. That is, the thickness of the steam-permeable portion 31B is required to be designed according to the container to be used and the object while ease with molding and advantageous effects of steam permeability are verified. In the case of a third variational example, it is preferable that the material of the hard container 3 be polycarbonate or the like that has high heat resistance and allows steam to permeate easily.

The formation of the steam-permeable portions 31B allows steam to enter the hard container 3 (inside of the blood processing filter 1) during autoclave sterilization, and enables easy sterilization in the blood processing filter 1. In this embodiment, the mode has been described where the steam-permeable portions 31B are formed at both the inlet-side container element 4 and the outlet-side container element 5. Alternatively, a mode may be adopted where the steam-permeable portion 31B is formed at one of the inlet-side container element 4 and the outlet-side container element 5.

The gas permeable coefficient of the steam-permeable portion 31B = the amount of gas permeation (volume) × thickness of steam-permeable portion (e.g., film 31a)/(pressure difference × permeation area × time). Accordingly, reduction in thickness of the steam-permeable portion 31B and increase in area can improve the steam permeability. Consequently, it is required to design the amount of gas (steam) permeation to be at least a certain amount (e.g., at least 0.50 g (measurement conditions: room temperature, 1 atm, and a day)) to achieve the thickness and area of the steam-permeable portion 31B that correspond to time during which autoclave sterilization is executed. An internal pressure occurs during filtration. Consequently, the strength for enduring the pressure is also required to be considered.

Meanwhile, the thickness of the other portion 31X integrally formed with the steam-permeable portion 31B has a higher priority in shape stability than in steam permeability. It is preferable that the thickness be larger than 500 $\mu$m. The upper limit may appropriately be defined according to the characteristics of the material to be used and moldability in processing.

In this embodiment, ribs 4h and 5h are provided between the respective steam-permeable portions 31B and the filter element 2. As a result, even if the internal pressure in the blood processing filter 1 becomes a negative pressure during filtration, the steam-permeable portion 31B is not pressed against the filter element 2. Consequently, it becomes unlikely to cause extension of filtering time and the like.

As to the method of integrally forming the steam-permeable portion 31B at a part or parts of one of or both the inlet-side container element 4 and the outlet-side container element 5, a corresponding portion of an injection molding mold may be configured to achieve narrowness so that only the portion concerned is made thin in the case of molding the container element through injection molding or the like. Alternatively, the thin-walled portion may be formed by grinding or polishing the molded container. It is better to make the corresponding portion of the injection molding mold to achieve narrowness because of reduction in steps and prevention of contamination with foreign bodies.

(About filter element)

The filter element 2 may be made of a filtering medium, such as a fibrous and porous medium, e.g., nonwoven fabric, woven fabric or the like, or a porous body having three-dimensional continuous reticulate pores, e.g., spongiform fabric. Examples of the materials include polypropylene, polyethylene, polyurethane, polyester and the like. The case where the filter element 2 is made of nonwoven fabric is particularly preferable in view of productivity.

The filter element 2 may be a single filter element, or made of a plurality of filter elements made of stacked sheet-shaped filter materials. In the case where the element is made of a plurality of filter elements, it is preferable that the element include a first filter element that be disposed upstream and mainly remove microaggregates, and a second filter element that be disposed downstream of the first filter element for removing undesirable components other than microaggregates.

For example, a filter material made of nonwoven fabric having fiber diameters ranging from several to several tens of micrometers is disposed on the inlet side as the first filter element for mainly removing aggregates, a filter material made of nonwoven fabric having fiber diameters ranging from 0.3 to 3.0 $\mu$m is disposed as the second filter element for removing undesirable components other than aggregates, and these materials can be used. Each of the first and second filter elements may be made of a plurality of types of filter materials having different fiber diameters. Alternatively, only one of these elements may be made of a plurality of types of filter materials.

For example, a first filter element made of nonwoven fabric having fiber diameters ranging from 30 to 40 $\mu$m and/or nonwoven fabric having fiber diameters ranging from 10 to 20 $\mu$m may be disposed on the upstream side, and a second filter element made of nonwoven fabric having fiber diameters ranging from 1.5 to 2.5 $\mu$m and/or nonwoven fabric having fiber diameters ranging from 0.5 to 1.8 $\mu$m on the downstream side of the first filter element, thereby forming the filter element 2. Nonwoven fabric having large fiber diameters and nonwoven fabric having small fiber diameters may be alternately arranged. It is however preferable that the nonwoven fabric having large fiber diameters be arranged on the upstream side.

(About compression of filter element by fitting portion and gripper of hard container)

As described above, the hard container 3 is a rectangular container having a predetermined thickness, and includes the inlet-side container element 4 and the outlet-side container element 5 which are arranged to clamp the filter element 2. The internal space 3s of the hard container 3 is separated by the filter element 2 into the inlet space 4s and the outlet space 5s. The filter element 2 has a filtering surface 2a that faces the inlet space 4s, a filtering surface 2b that faces the outlet space 5s, and an end surface 2c along the peripheries of the pair of filtering surfaces 2a and 2b.

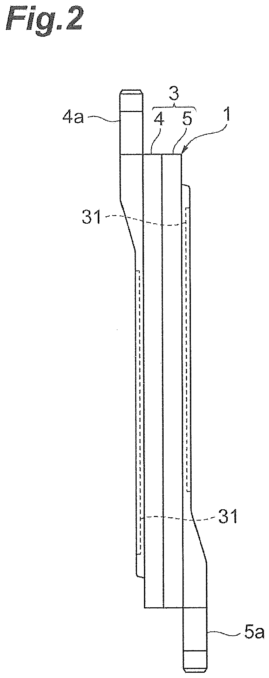

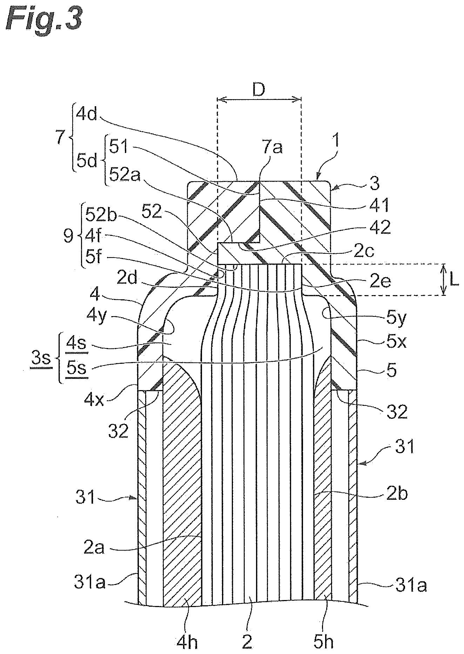

The inlet-side container element 4 is a rectangular member obtained by cutting and substantially halving the hard container 3 in the thickness direction (see Figure 2). The outlet-side container element 5 is a rectangular member that is the rest of the hard container 3. The periphery of the inlet-side container element 4 is provided with an inlet-side fitting portion 4d (see Figure 3). The periphery of the outlet-side container element 5 is provided with an outlet-side fitting portion 5d that is fitted with the inlet-side fitting portion 4d. The inlet-side fitting portion 4d and the outlet-side fitting portion 5d have a male and female relationship, and surround the end surface 2c of the filter element 2 and are fitted to each other to form a fitting portion 7. In the following description, the fitting portion 7 is described further in detail using an example where the inlet-side fitting portion 4d serves as a male side and the outlet-side fitting portion 5d serves as the female side. Alternatively, the male and female relationship may be inverted.

The fitting portion 7 is described further in detail. The periphery of the inlet-side container element 4 is provided with the convex inlet-side fitting portion 4d. Meanwhile, the periphery of the outlet-side container element 5 is provided with a wall portion 52 that comes into contact and is engaged with an internal peripheral surface 42 of the inlet-side fitting portion 4d, and a base portion 51 that comes into contact with a distal end portion 41 of the inlet-side fitting portion 4d. The outlet-side fitting portion 5d is formed of an external surface 52a and the base portion 51 of the wall portion 52. A contact site between the inlet-side fitting portion 4d and the outlet-side fitting portion 5d is a sliding contact portion 7a of the fitting portion 7. The sliding contact portion 7a may be subjected to bonding or adhesion using ultrasonic bonding or adhesive.

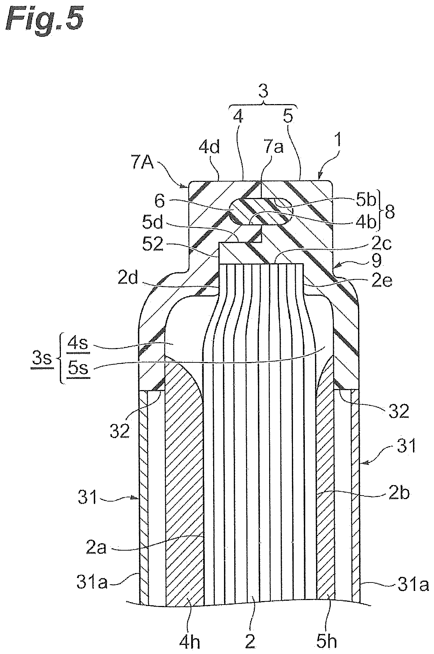

As a variational example of the fitting portion 7, for example, a fitting portion 7A shown in Figure 5 may be adopted. The fitting portion 7A is provided with a resin flow path 8 at the sliding contact portion 7a between the inlet-side fitting portion 4d and the outlet-side fitting portion 5d, and allows adhesion using resin with which the resin flow path 8 is filled. The resin flow path 8 is a cavity made of a groove 4b and groove 5b that have a U-shaped section and are provided at the inlet-side fitting portion 4d and the outlet-side fitting portion 5d, respectively. At least one of the sliding contact portion 7a is bonded in a belt-shaped manner along the entire periphery of the fitting portion 7A by the inside of the resin flow path 8 being filled with melt resin 6. With such a belt-shaped bonding along the entire periphery of the fitting portion 7, the hard container 3 is sealed, which improves the airtightness and liquid-tightness. The resin flow path 8 has a through-hole (not shown) that communicates with the inside of the hard container 3 or the outside of the hard container 3. The number of through-holes may be one or more.

In this variational example, both the groove 4b and the groove 5b are provided to show the example of the mode of forming the resin flow path 8. Alternatively, the resin flow path 8 may be formed of the one groove 4b (or groove 5b). As to the blood processing filter 1 including the fitting portion 7A according to this variational example, the example of the mode of forming the steam-permeable portion 31 made of the film 31a having steam permeability is described. As shown in Figure 4, the steam-permeable portion 31A (see Figure 4) may be formed by providing a flexible resin having steam permeability instead of the film 31a. In the case where the materials themselves of the inlet-side container element 4 and the outlet-side container element 5 are materials that allow steam to permeate easily, it may be sometimes sufficient to create partially the thin-walled portion to further improve the steam permeability of the inlet-side container element 4 and outlet-side container element 5 themselves. In such cases, the thickness of the thin-walled portion may be a thickness ranging from 50 to 500 $\mu$m, inclusive, to improve the steam permeability.

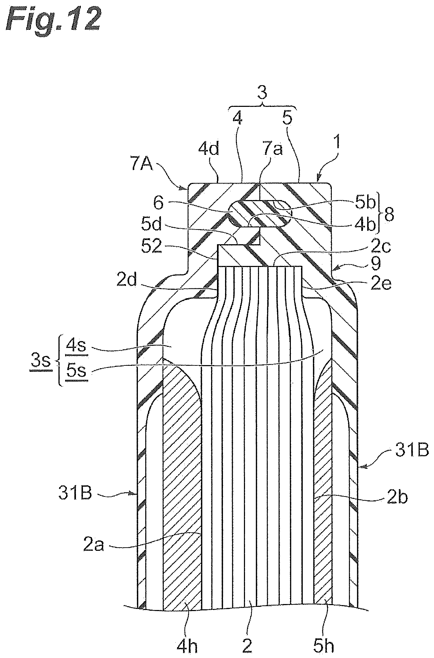

As shown in Figure 12, in the blood processing filter 1 that includes the steam-permeable portion 31B according to the second variational example, the fitting portion 7A according to the variational example may be provided.

As shown in Figure 3, the inside of the fitting portion 7 is provided with a gripper 9 that clamps and compresses outer edge portions 2d and 2e of a pair of filtering surfaces 2a and 2b, which are the front and back of the filter element 2. The gripper 9 is formed of an inlet-side compression portion 4f formed at the inlet-side container element 4, an outlet-side compression portion 5f formed at the outlet-side container element 5, and an internal surface of the wall portion 52 formed at the outlet-side container element 5. The internal surface of the wall portion 52 serves as a close contact portion 52b that comes into close contact with the end surface 2c of the filter element 2.

The close contact portion 52b according to this embodiment is formed of the internal surface of the wall portion 52 provided for the outlet-side container element 5. However, for example, in the case where the male and female relationship between the inlet-side fitting portion 4d and the outlet-side fitting portion 5d that form the fitting portion 7 (or fitting portion 7A) is inverted, the close contact portion may be formed of the internal surface of the wall portion provided for the inlet-side container element 4. Instead of the fitting portion 7 (or fitting portion 7A), wall portions that are to be pressed against each other may be provided for both the inlet-side container element 4 and the outlet-side container element 5, the internal surfaces of both the wall portions cooperate with each other to be in close contact with the end surface 2c of the filter element 2, thereby forming the close contact portion.

The inlet-side compression portion 4f is made of a step that is the periphery of the inlet-side container element 4 curved toward the inner surface side (outlet-side container element 5 side) and bent to the outside. Likewise, the outlet-side compression portion 5f is made of a step that is the periphery of the outlet-side container element 5 curved toward the inner surface side (inlet-side container element 4 side) and bent to the outside. The inlet-side compression portion 4f and the outlet-side compression portion 5f clamp and compress the outer edge portions 2d and 2e of the pair of filtering surfaces 2a and 2b that are the front and back of the filter element 2.

The gripper 9 allows the inlet-side compression portion 4f and the outlet-side compression portion 5f to compress a range with a width L ranging from 1 to 5 mm from the outermost edge of the filter element 2. Such a configuration with the gripper 9 having the width L more than 1 mm can prevent the filter element 2 from being apart from the gripper 9, and prevent insufficient removal from occurring while the filter element 2 is pressed. The configuration where the width L of the gripper 9 that forms an area unusable for filtration is smaller than 5 mm achieves easy design within a range allowable in blood processing efficiency or economically.

In an inner range surrounded by the inlet-side compression portion 4f on the inner surface side of the inlet-side container element 4, a plurality of convex ribs 4h are provided. In an inner range surrounded by the outlet-side compression portion 5f on the inner surface side of the outlet-side container element 5, a plurality of convexes 5h are provided. The ribs 4h of the inlet-side container element 4 are pressed against the filtering surface 2a of the filter element 2, and secure the inlet space 4s between the inner surface and the filtering surface 2a of the inlet-side container element 4. Likewise, the ribs 5h of the outlet-side container element 5 are pressed against the filtering surface 2b of the filter element 2, and secure the outlet space 5s between the inner surface and the filtering surface 2b of the outlet-side container element 5.

As described above, in the blood processing filter 1 according to this embodiment, the pair of filtering surfaces 2a and 2b of the filter element 2 is clamped by the ribs 4h and 5h, and partially compressed, thereby effectively and stably securing the inlet space 4s and the outlet space 5s. In this embodiment, the rib 4h of the inlet-side container element 4 has a larger height than the rib 5h. As a result, the inlet space 4s is secured larger than the outlet space 5s. The heights of the ribs 4h and 5h may be the same. On the contrary, the rib 5h may be higher.

The thickness D of the filter element 2 at the gripper 9 is required to be compressed to be 0.05 to 0.5 times as thick as the original thickness D0, and D/D0 = 0.05 to 0.5. Here, since the filter element 2 cannot be compressed to have a density less than that of the original resin, in actuality it is difficult to make the filter have a thickness smaller than D/DO = 0.05. On the other hand, if the thickness is larger than D/D0 = 0.5, the filter element 2 is not in a compressed state with a high density. A risk occurred that undesirable components flow over the outer edge portions 2d and 2e of the filter element 2 and result in a side leakage (side flow). Consequently, the performance of removing undesirable components, such as leukocytes, decreases. The tendency is due to a high temperature during steam sterilization at high temperature and high pressure causing contraction of the container, and becomes significant. That is, it is important to have the filter element 2 with the thickness D, which is achieved by compressing the element to have a density close to the density of the original resin. As a result, the performance of removing undesirable components, such as leukocytes, can be improved. Thus, for example, in the case where the original thickness DO of the filter element 2 is 10 mm, it is required to compress the gripper 9 to have a thickness D of about 0.5 to 5 mm. In order to compress the gripper 9 to have the thickness D 0.05 to 0.5 times as thick as the original thickness D0, the molding die is adjusted such that the distance of the gripper 9, that is, the distance between the opposite surfaces of the inlet-side compression portion 4f and the outlet-side compression portion 5f should have a thickness 0.05 to 0.5 times as thick as the original thickness D0. In view of compatibility between easy manufacturability and performance of removing undesirable components, such as leukocytes, it is preferable that D/DO = 0.07 to 0.45, and it is further preferable that D/D0 = 0.1 to 0.4.

Next, the thickness D0 of the filter element 2 in the case of verifying the molded blood processing filter 1 after molding is described. When the molded blood processing filter 1 after molding is taken apart and the thickness of a portion which is not clamped is measured, the maximum value D1 of the thickness is a value within an error range, which is several percent, with reference to the original thickness D0 before clamping by the gripper 9, and the maximum value D1 can be substantially regarded as the original thickness D0. More specifically, the maximum value D1 is the maximum value of the thicknesses at five points selected from portions which are other than portions of the filter element 2 deformed by the convexes in the hard container 3, such as the gripper 9 and the rib 4h and the rib 5h, and are at and around the center of the filter element 2 and do not have a concave or the like, and have an unchanged form. The maximum value D1 can be regarded as the original thickness D0 before clamping by the gripper 9.

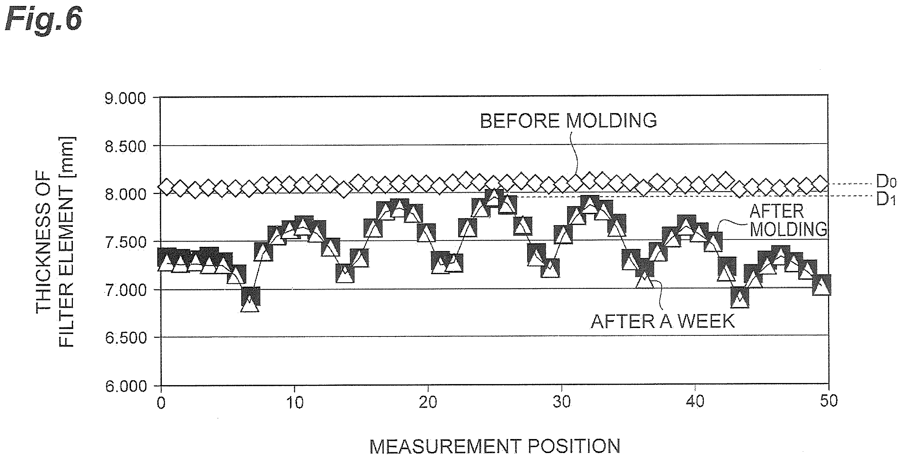

An experimental result supporting this verification is described with reference to Figure 6. Figure 6 is a graph comparing the thickness of a section of the filter element 2 (polyester nonwoven fabric) before and after molding of the blood processing filter 1 (the blood processing filter 1 after molding is taken apart). As shown in Figure 6, in the case of this blood processing filter 1, the difference between the original thickness D0 and the maximum value D1 was approximately 3% of the original thickness D0. That is, it can be verified that the difference between the original thickness D0 of the filter element 2 and the maximum value D1 of the filter element 2 obtained by taking apart the blood processing filter 1 after molding is approximately several percent of the original thickness D0. As to the time of measurement after taking apart, comparison between thickness measurement results immediately after taking apart and those having been left for one week after taking apart show little variation. Consequently, it was verified that time elapsed from taking apart to measurement has little effect on thickness measurement results.

According to the above results, in the case where it is easier to detect the maximum value D1 of the filter element 2 obtained by taking apart the blood processing filter 1 after molding than to grasp the original thickness D0 before molding, the value of D/D0 can be grasped by detecting the maximum value D1 instead of the thickness D0 and regarding the maximum value D1 as the original thickness D0. More specifically, D1 should be equal to D0 or smaller by several percent (i.e., $D0 \geq D1$). Consequently, when the thickness D is 0.5 or smaller times larger than the maximum value D1 (i.e., $D/D1 \leq 0.5$), the thickness D is equal to or smaller than a value that is 0.5 times as large as the original thickness D0 (i.e., $D/D0 \leq 0.5$). On the other hand, since the filter element 2 cannot be compressed to have a density that is equal to or smaller than the original resin, it is difficult to believe that the lower limit value is smaller than 0.05. Consequently, it is difficult to believe that the thickness D is smaller than 0.05 of the original thickness D0 while the thickness D is equal to or larger than 0.05 of the maximum value D1. That is, when D/D1 = 0.05 to 0.5, it can be regarded that D/D0 = 0.05 to 0.5.

The difference between the maximum value D1 and the original thickness D0 is approximately several percent of the original thickness D0, and can thus be considered to be substantially ignored. However, even in the case of D/D1 > 0.5, the error of several percent does not negate the possibility D/D0 ≤ 0.5 if D0 can be actually detected.

Next, the case of D/D0 = 0.07 to 0.45 and the case of D/D0 = 0.1 to 0.4 are described. The value of D/D0 can physically be less than the lower limit values of 0.07 and 0.1. Thus, the lower limit value of D/D1 at which the lower limit value of D/D0 is securely 0.07 and 0.1 or more. Here, provided that the difference between the maximum value D1 and the original thickness D0 is a value, e.g., 10%, which is significantly larger than that in the above experiment (see Figure 6), D/D1 = 0.078 (= 0.07/0.9) in the case of D/D0 = 0.07, and D/D1 = 0.11 (= 0.1/0.9) in the case of D/D0 = 0.1. That is, in the case of at least D/D1 = 0.078 to 0.45, it is not improbable to regard D/D0 = 0.07 to 0.45. In the case of D/D1 = 0.11 to 0.4, it is not improbable to regard D/D0 = 0.1 to 0.4.

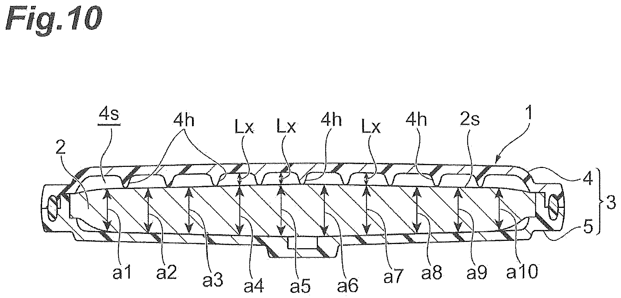

On the other hand, it is preferable that the coefficient of variation value (CV value) of the thickness of the filter element 2 be 6% or less. As shown in Figure 10, the filter element 2 is fixed by the ribs 4h provided to fix the filter element 2 at a predetermined position in the inlet space 4s and the outlet space 5s. However, since the element is compressed during fixation, the thickness is prone to be uneven. This is because, even if the repulsive power to compression during fixation is equivalent, deformation at the deformable center portion becomes large, and the thickness becomes uneven between the center portion and the peripheral portion. In particular, in the case of using the ultrasonic bonding method to bond the container, only the portion corresponding to the bond portion of the container, i.e., the outermost periphery of the container, is pressed. Consequently, the thicknesses of the filter element at the central portion of the filter and the peripheral portion of the filter are prone to be uneven. Here, if the thicknesses of the central portion of the filter and the peripheral portion of the filter are uneven, the unevenness causes drift, and possibility of problems. The drift (drifting flow) is a phenomenon that blood selectively passes through a specific portion, and a portion resistant to the flow occurs.

In order to solve the above problems, in the blood processing filter 1 according to this embodiment, the thicknesses (a1, a2, ..., a10) of the filter element 2 are measured on at least three points between the rib 4h and the rib 4h and between the rib 4h and the peripheral portion, and the coefficient of variation value (CV value), which is the division of the standard deviation by the average value, is set to 6% or less. As a result, the drift can be prevented, and the blood processing filter 1 where the entire filter element 2 can be effectively used can be achieved. Here, the thickness of the filter element 2 may be measured on at least three points. It is preferable that the number of points be at least five, and between all the ribs 4h and between the ribs 4h and the peripheral portion be measured.

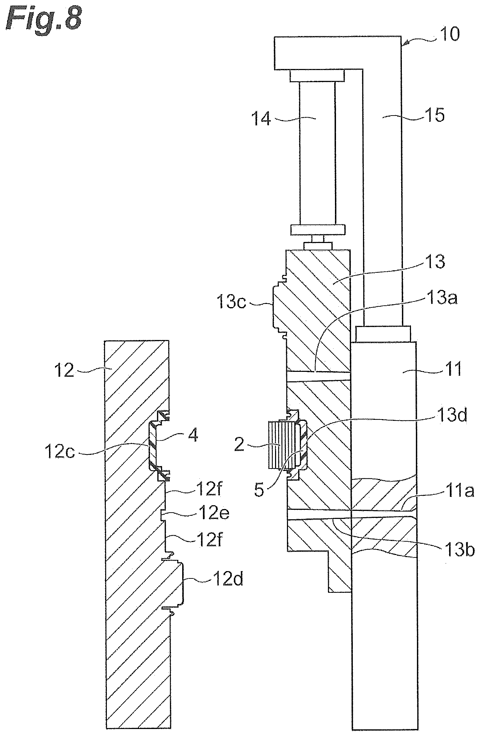

Furthermore, it is preferable that the coefficient of variation value (CV value) of the distance Lx from the inlet-side container element 4 to the inlet-side surface 2a of the filter element 2 be 10% or less at the inlet space 4s. As shown in Figure 8, the filter element 2 is fixed by the ribs 4h provided to fix the filter element 2 at a predetermined position in the inlet space 4s and the outlet space 5s. However, since the filter element 2 is compressed during fixation, the thickness is prone to be uneven. Consequently, the distance between the inlet-side container element 4 and the inlet-side surface 2s of the filter element 2 is also prone to be uneven. In particular, in the case of using the ultrasonic bonding to bond the container, only the portion corresponding to the bond portion of the container, i.e., the periphery of the container, is pressed. Consequently, the thicknesses of the filter element 2 at the central portion of the filter and the peripheral portion of the filter are prone to be uneven.

In order to solve the above problems, in the blood processing filter 1 according to this embodiment, the distance Lx from the inlet-side container element 4 and the inlet-side surface 2s of the filter element 2 in the inlet space 4s is measured, corresponding to the portions measured as the thickness of the filter element 2 between the rib 4h and the rib 4h and between the rib 4h and the peripheral portion, and the coefficient of variation value (CV value: coefficient of variation value), which is the division of the standard deviation by the average value, is set to 10% or less. As a result, blood and the like entering from the inlet uniformly spreads into the inlet space 4s, and the filter element 2 can be effectively used.

Next, an example of a method for manufacturing the blood processing filter 1 is described. The manufacturing method is described below using the blood processing filter 1 where the resin flow path 8 is formed at the sliding contact portion 7a between the inlet-side fitting portion 4d and the outlet-side fitting portion 5d. The blood processing filter 1 where the resin flow path 8 is not formed at the sliding contact portion 7a is basically according to analogous steps. First, an injection molding machine 10 used for the manufacturing method is described.

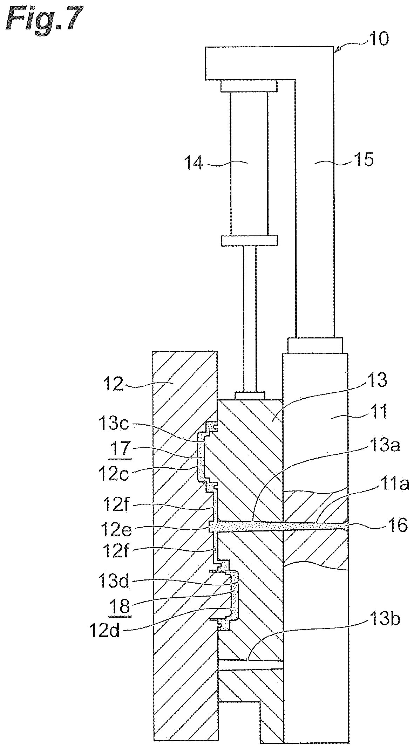

As shown in Figure 7, the injection molding machine 10 includes a fixed die 11, a movable die (mold) 12, and a slidable die (mold) 13. The fixed die 11 is fixed to a fixed platen (not shown) of the injection molding machine 10. A platform 15 that includes a cylinder 14 for sliding is provided on the upper surface of the fixed die 11. The cylinder 14 that is hydraulically or pneumatically moved is coupled onto the upper surface of the slidable die 13. The slidable die 13 is configured to be slidable and movable in a vertical direction while the state of being in close contact with a side surface of the fixed die 11 is kept.

The movable die 12 is attached to a movable platen (not shown) that is horizontally movable. The movable platen is configured to be movable in a manner close to but apart from the fixed die 11 by a mold opening and closing device (not shown) of the injection molding machine 10. The movable die 12 is configured to be movable between a mold closing position of being in close contact with the slidable die 13, and a mold opening position of being apart from the slidable die 13.

The fixed die 11 is provided, at its center, with a sprue 11a for guiding melt resin 16 to be ejected from an injector (not shown) attached to the fixed die 11. The slidable die 13 is provided with a central sub-sprue 13a that continuously communicates with the sprue 11a when the die is at a lower position, and with a lower sub-sprue 13b that continuously communicates with the sprue 11a when the die is moved to an upper position.

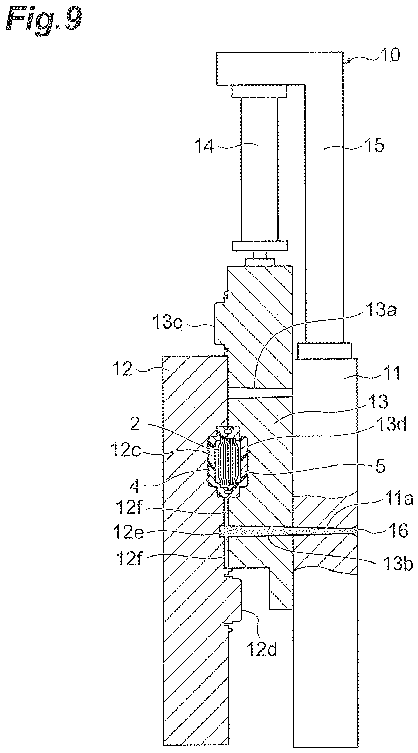

A mold closing surface of the slidable die 13 is provided with a male mold 13c and a female mold 13d at vertically symmetric positions with respect to the central sub-sprue 13a. The male mold 13c is for molding the inner surface 4y of the inlet-side container element 4, and the female mold 13d is for molding the outer surface 5x of the outlet-side container element 5. On the other hand, the mold closing surfaces of the movable die 12 are provide with a female mold 12c and a male mold 12d facing the respective male mold 13c and female mold 13d when the slidable die 13 is at the lower position. The female mold 12c is for molding the outer surface 4x (see Figure 3) of the inlet-side container element 4, and the male mold 12d is for molding the inner surface 5y of the outlet-side container element 5. As shown in Figures 8 and 9, the female mold 12c on the movable die 12 side is configured to face the female mold 13d on the slidable die 13 side when the slidable die 13 is at the upper position.

As shown in Figure 7, when the slidable die 13 is at the lower position and the movable die 12 is mold-closed therewith, a pair of gaps 17 and 18 surrounded by the male molds 12d and 13c and the female molds 13d and 12c are formed between the slidable die 13 and the movable die 12. At this time, the central sub-sprue 13a of the slidable die 13 is configured to communicate with these gaps through a runner 12e formed from the edge portions of the female molds 13d and 12c at the movable die 12 and through a pair of gates 12f. As shown in Figure 9, when the slidable die 13 is at the upper position and the movable die 12 is mold-fit therewith, the female molds 13d and 12c of the slidable die 13 and the movable die 12 match with each other, and the lower sub-sprue 13b and the runner 12e communicate with the edge portions of these female molds 13d and 12c through the gates 12f.

In order to mold the blood processing filter 1 using the injection molding machine 10, first, as shown in Figure 7, the cylinder 14 is elongated to place the slidable die 13 at the lower position. Then, the movable platen of the injection molding machine 10 is moved on the fixed platen side, and mold-closes the slidable die 13 and the movable die 12. In this state, the central sub-sprue 13a of the slidable die 13 communicates with the sprue 11a of the fixed die 11, and the pair of gaps 17 and 18 are formed between the slidable die 13 and the movable die 12.

Next, the melt resin 16 is injected from the injector attached to the fixed platen, and the melt resin 16 is guided to both the gaps 17 and 18 through the sprue 11a of the fixed die 11, the central sub-sprue 13a of the slidable die 13, the runner 12e, and the gates 12f, and these gaps 17 and 18 are filled therewith. Thus, the male and female pair of inlet-side container element 4 and outlet-side container element 5 are formed in the respective gaps 17 and 18 (container element molding step). At this time, the steam-permeable films 31a are preliminarily disposed in the mold and insertion molding is performed, which can fabricate the inlet-side container element 4 and the outlet-side container element 5 where the steam-permeable films 31a are disposed. At this time, if the mold of the injection molding machine 10 is configured to achieve a partially thin portion, the inlet-side container element 4 and the outlet-side container element 5 that are provided with steam-permeable portions 31B can be fabricated.

After the male and female pair of inlet-side container element 4 and outlet-side container element 5 is cooled and solidified, the movable die 12 and the slidable die 13 are mold-opened by the mold opening and closing device and separated as shown in Figure 8. Then, the male molds 13c and 12d are separated from the inlet-side container element 4 and the outlet-side container element 5, and the inlet-side container element 4 and the outlet-side container element 5 are left on the female molds 12c and 13s. In the mold opening, the resin portion solidified in the sprue 11a, the sub-sprue 13a, the runner 12e, the gates 12f and the like is pushed out of and dropped from the molds. The thus obtained inlet-side container element 4 and outlet-side container element 5 are provided with the inlet-side fitting portion 4d and the outlet-side fitting portion 5d, which are brought into contact with each other to form the fitting portion 7, along the inner edge of the rectangular external shape.

Next, the filter element 2 made of polyester nonwoven fabric is inserted into the outlet-side container element 5, and subsequently the cylinder 14 is retracted to move the slidable die 13 to the upper position (inserting step). Then, the female mold 13d of the slidable die 13 and the female mold 12c of the movable die 12 face with each other, the inlet-side container element 4 and the outlet-side container element 5 left on the female molds 13d and 12c are brought into a state of facing each other. At this time, the lower sub-sprue 13b of the slidable die 13 is placed to communicate with the sprue 11a of the fixed die 11.

In this state, the movable die 12 is moved toward the slidable die 13, and these dies are brought into contact with each other and mold-closed as shown in Figure 9 (fitting step). Thus, the fitting surfaces of the inlet-side fitting portion 4d and the outlet-side fitting portion 5d are fit with each other to form the hollow resin flow path 8, which includes the groove 4b and the groove 5b of the sliding contact portion 7a. The fitting step is a mode of the joining step.

The resin flow path 8 has a through-hole, and communicates with the gates 12f through the through-hole. The gates 12f further communicate with the sub-sprue 13b through the runner 12e. In this state, the melt resin 16 serving as the melt resin 6, with which the resin flow path 8 is to be filled (see Figure 5), is ejected from the injector. The melt resin 16 flows through the sprue 11a of the fixed die 11, the sub-sprue 13b, the runner 12e, the gates 12f, and the through-hole, and the resin flow path 8 is filled with this resin as the melt resin 6. Thus, the inlet-side container element 4 and the outlet-side container element 5 are bonded to each other at the periphery of the fitting portion 7 with the melt resin 6 (fixing step). As described above, according to the method of filling with the melt resin 6 after formation of the resin flow path 8, easy control of the amount of resin is achieved.

The slidable die 13 and the movable die 12 are mold-opened again by the mold opening and closing device after the melt resin 6 is cooled and solidified, and the inlet-side container element 4 and the outlet-side container element 5 are fit and bonded to each other, and thus the blood processing filter 1, which is finished as a completely sealed molded product, is obtained. The resin portion solidified in the sprue 11a, the sub-sprue 13a, the runner 12e, the gates 12f and the like is pushed out of and dropped from the molds.

After the thus completed blood processing filter 1 is taken out, the cylinder 14 is elongated again to place the slidable die 13 at the lower position. Then, the movable die 12 and the slidable die 13 are mold-closed, and transition is made to a molding step for the next product. The series of steps as described above is repeated, thereby allowing the blood processing filters 1 to be successively molded. Furthermore, the molding step includes vertical sliding of the slidable die 13, mold-closing and mold-opening through forward and backward movement of the movable die 12, and injection of the melt resin 16. Consequently, all the steps can be easily automated. The blood processing filter 1 can therefore be mass-produced.

Thus, only through use of the movable die 12 and the slidable die 13, which serve as the set of molds, and the injection molding machine 10, processes from injection molding of the inlet-side container element 4 and the outlet-side container element 5, and internal inserting of the filter element 2, and to bonding of the inlet-side container element 4 and the outlet-side container element 5, which are male and female container elements, can be consecutively performed in a single step, and even what is completely sealed can be molded.

As described above, the blood processing filter 1 according to this embodiment includes the sheet-shaped filter element 2, and the hard container 3 that includes the inlet-side container element 4 and outlet-side container element disposed to clamp the filter element 2, and the internal space 3s separated by the filter element 2 into the inlet space 4s and the outlet space 5s. The filter element 2 includes a pair of filtering surfaces 2a and 2b disposed on the inlet space 4s side and the outlet space 5s side, respectively. The inlet-side container element 4 and the outlet-side container element 5 are provided with the gripper 9 which clamps and compresses the outer edge portions 2d and 2e of the pair of filtering surfaces 2a and 2b. The filter element 2 is compressed to allow the portion clamped by the gripper 9 to have a thickness 0.05 times or more and 0.5 times or less larger. The hard container 3 is provided with steam-permeable portions 31, 31A and 31B having steam permeability.

The hard container 3 of the blood processing filter 1 is thus provided with the steam-permeable portions 31, 31A and 31B having steam permeability. Consequently, sterilization in an autoclave can be easily executed. The inlet-side container element 4 and the outlet-side container element 5 are provided with the gripper 9 which clamps and compresses the outer edge portions 2d and 2e of the pair of filtering surfaces 2a and 2b. The filter element 2 is compressed by the gripper 9 to have a thickness 0.05 to 0.5 times larger. As a result, the side leakage (side flow) where undesirable components flow over the outer edge portions 2d and 2e of the filter element 2 without being filtered can be prevented.

The gripper 9 according to this embodiment includes the close contact portion 52b that is to be in close contact with the end surface 2c of the filter element 2. Accordingly, the gripper 9 is positioned to be in close contact with the end surface 2c of the filter element 2. Consequently, the gripper 9 is resistant to deviation. The resistance is advantageous in high-density compression of the outer edge portions 2d and 2e of the pair of filtering surfaces 2a and 2b against each other. As a result, even if the region of the outer edge portions 2d and 2e compressed by the gripper 9 is configured to be small, compression can be securely performed by the gripper 9. Consequently, the risk of incomplete removal of undesirable components can be easily prevented without reducing the blood processing efficiency.

Furthermore, in the blood processing filter 1, the gripper 9 compresses the range with the width L ranging from 1 to 5 mm, inclusive, from the outermost edge of the filter element 2, as the outer edge portions 2d and 2e. Consequently, the risk of incomplete removal of undesirable components can be easily prevented without reducing the blood processing efficiency. Furthermore, the filter element 2 can be prevented from being apart from the gripper 9, and easy designing can be achieved within allowable range in view of blood processing efficiency or economically.

Furthermore, the inlet-side container element 4 and the outlet-side container element 5 of the blood processing filter 1 are provided with fitting portions 7 and 7A that surround the end surface 2c of the filter element 2 for fitting. As the fitting portions 7 and 7A are provided, the inlet-side container element 4 and the outlet-side container element 5 can be easily aligned. Furthermore, the inlet-side container element 4 and the outlet-side container element 5 can be easily joined.

Furthermore, the fitting portions 7 and 7A include the inlet-side fitting portion 4d provided for the inlet-side container element 4, and the outlet-side fitting portion 5d provided for the outlet-side container element 5. At least a part of the sliding contact portion 7a between the inlet-side fitting portion 4d and the outlet-side fitting portion 5d can be bonded with melt resin in a belt-shaped manner over the entire peripheries of the fitting portions 7 and 7A. In this case, airtightness and liquid-tightness can be improved.

Furthermore, a mode can be adopted where the sliding contact portion 7a of the fitting portion 7A is provided with the resin flow path 8, and the inside of the resin flow path 8 is filled with the melt resin 6 and the sliding contact portion 7a is bonded. In this case, the melt resin 6 is resistant to deviating from the sliding contact portion 7a, and has no possibility of entering the inside of the hard container 3.

Furthermore, in the blood processing filter 1, the hard container 3 is made of resin with a tensile elastic modulus of at least 1 GPa at room temperature, which can prevent deformation during filtration.

Furthermore, the blood processing filter 1 is configured so that the coefficient of variation value (CV value) of the thickness of the filter element 2 is 6% or less. As a result, the drift can be prevented, and the entire filter element 2 can be effectively used.

Furthermore, in the blood processing filter 1, the coefficient of variation value of the distance from the inlet-side container element 4 to the inlet-side surface 2s of the filter element 2 in the inlet space 4s is set to 10% or less. As a result, blood and the like entering from the inlet uniformly spreads into the inlet space 4s, and the filter element 2 can be effectively used.

The method for manufacturing the blood processing filter 1 includes: a container element molding step of injection-molding the inlet-side container element 4 with one mold between the male mold 13c of the slidable die 13 and the female mold 12c of the movable die 12 and the outlet-side container element 5 with the other mold, and insert-molding the film 31a having steam permeability to form the steam-permeable portion 31; and a inserting step of inserting the filter element 2 into the inlet-side container element 4 or the outlet-side container element 5.

Furthermore, the method includes: a fitting step (joining step) of mold-closing (mold-fitting) the movable die 12 and the slidable die 13 which serve as a set of molds and fitting the inlet-side container element 4 and the outlet-side container element 5; and a fixing step of bonding the inlet-side container element 4 and the outlet-side container element 5 with the melt resin 6.

According to this manufacturing method, the fitting step of mold-closing the movable die 12 and the slidable die 13 and fitting the inlet-side container element 4 and the outlet-side container element 5, thereby allowing the inlet-side container element 4 and the outlet-side container element 5 to be pressed against each other by a strong power of, e.g., several tens of tf (ton-force). Ultrasonic bonding achieves approximately several hundreds kgf (kilogram-force), and the inlet-side container element 4 and the outlet-side container element 5 can be fixed to each other by ultrasonic bonding. Alternatively, according to this embodiment that executes the fixing step after the fitting step, the filter element 2 can be compressed to a high density.

In particular, the manufacturing method can compress the filter element 2 to a higher density than the case of simply performing ultrasonic bonding. Consequently, a leak from the bonded portion of the blood processing filter 1 can be effectively prevented from occurring. Furthermore, the need to fix the inlet-side container element 4 and the outlet-side container element 5 to each other while excessively pressing against each other is negated. Consequently, the bonded portion between the inlet-side container element 4 and the outlet-side container element 5 can be prevented from causing a crack.

The method for manufacturing the blood processing filter 1 according to this embodiment described above can effectively produce the aforementioned blood processing filter 1.

Although the present invention has thus been described with reference to the embodiments, the present invention is not limited to the embodiments. For example, the inlet-side container element 4 and the outlet-side container element 5 may be fitted to each other, and subsequently the hard container 3 and the filter element 2 may be integrally bonded to each other using high frequency bonding to thereby obtain the blood processing filter 1. After the hard container 3 is formed, the resin flow path 8 may be filled with the melt resin 6 according to another method to obtain the blood processing filter 1. An example of the other method may be a method of mounting the filter element 2, and preliminarily filling, with the melt resin 6, at least one of the groove 4b and the groove 5b, which are to be the resin flow path 8, and then performing mold closing.

As to the blood processing filter 1 according to this embodiment, the mode provided with the fitting portions 7 and 7A have been described. Alternatively, a mode may be adopted where the fitting portions 7 and 7A are not provided, for example, the inlet-side contact portion (not shown) and the outlet-side contact portion (not shown) that allow the sliding contact portion 7a to be form a plane. That is, the inlet-side container element and the outlet-side container element do not have a male-female relationship. Furthermore, in the case of forming the blood processing filter according to this mode, without execution of the fitting step after the inserting step, the inlet-side container element and the outlet-side container element may be placed to face each other, brought into contact with each other in a state of clamping the filter element, and subsequently the fixing step may be executed.

**Examples**

The present invention will now be described in further detail below by way of examples.

(Filtering performance evaluation on whole blood)

A whole blood preparation adjusted as follows was used for filtering performance evaluation. 2100 mL of pig whole blood was collected and mixed into a blood bag containing 320 mL of anticoagulant CPD, and relatively coarse aggregates generated during blood collection were removed by filtration with a preprocess filter other than the blood processing filters of Examples and Comparative Examples of blood processing filters, which will be described later. The preprocess filter has a configuration where twelve sheets with an average value of pore diameters of 60 $\mu$m and a weight per unit area of 50 g/m$^2$, eight sheets with an average value of pore diameters of 50 $\mu$m and a weight per unit area of g/m$^2$, and eight sheets with an average value of pore diameters of 50 $\mu$m and a weight per unit area of 30 g/m$^2$ are stacked from the upstream side in this order, and accommodated in a container made of a hard resin with a filtering area of 45 cm$^2$. The blood from which the coarse aggregates in blood collection had been removed by the preprocess filter during blood collection was divided into 460 mL-portions and injected into respective bags on the day of blood collection. The thus obtained whole blood preparations were left at room temperature, and filtered with the blood filters of Examples and Comparative Examples at room temperature on the day of the blood collection. The filtering time from the start to the end of filtration was measured, and regarded as the filtering time. The leukocyte removing performance was calculated by the following Equation (1).

$$\text{Leukocyte removing performance} = \text{Log (concentration of leukocyte before filtering (cells/}\mu\text{L)/concentration of leukocyte after filtration (cells/}\mu\text{L))...(1)}$$

The concentrations of leukocyte before and after filtering were measured using an automatic blood cell analyzer SF3000 (Sysmex Corporation).

(Autoclave sterilization performance evaluation)

What includes a blood filter and a bioindicator inserted therein was fabricated, and stoppers made of PVC were used for the inlet of the inlet-side container element and the outlet of the outlet-side container element, thereby preventing steam from entering through the inlet or the outlet during sterilization. The sterilization was executed at 123°C for 30 minutes, and the performance of sterilization was evaluated according to presence or absence of cultured bacteria.

[Example 1]

The blood processing filter according to Example 1 was fabricated using the method of manufacturing the blood processing filter 1 according to the aforementioned embodiment. That is, the inlet-side container element was formed by the one mold and the outlet-side container element was formed by the other mold, and subsequently the blood processing filter element was loaded into the outlet-side container element, and the female and male inlet-side container element and outlet-side container element were brought into contact and fitted with each other by moving the mold. Subsequently, the blood processing filter was fabricated using the method of injecting the melt resin through the flow path of the mold formed at the periphery of the fitting portion of the inlet-side container element and the outlet-side container element into the resin flow path of the hard container and of bonding the entire periphery of the fitting portion.

The material of the hard container was polycarbonate resin, the distance of the gripper, that is, the thickness D of the filter element at the gripper was set to 3.6 mm, the width of the gripper, in other words, the width L of the outer edge portion (the outer edge portion of the filtering surface) of the filter element compressed by the gripper was set to 5 mm, and the effective filtering area was 46 cm$^2$. A film (having steam permeability) that allows steam to pass was provided to have an area of 13 cm$^2$ for the inlet-side container element. A film having steam permeability was provided to have an area of 16 cm$^2$ for the outlet-side container element. The steam-permeable portion was created for each. Here, a film made of polyvinyl chloride having a thickness of 0.2 mm was used as a film having steam permeability. The original thickness D0 of the filter element was 9.1 mm. The compressibility ratio by the gripper was 39.6% (compressed to 0.396 times). Polyester nonwoven fabric was stacked according to the following configuration for use as the filter element.

Polyester nonwoven fabric 1 (the average fiber diameter was 12 $\mu$m, and the weight per unit area was 30 g/m$^2$)     6 sheets

(continued)

| Polyester nonwoven fabric 2 (the average fiber diameter was 1.6 $\mu$m, and the weight per unit area was 66 g/m$^2$) | 2 sheets |
| Polyester nonwoven fabric 3 (the average fiber diameter was 1.2 $\mu$m, and the weight per unit area was 30 g/m$^2$) | 32 sheets |

The result of an experiment through use of the thus created blood processing filter is shown in Table 1. The leukocyte removing performance was 1.44, which showed high performance. Live bacteria after sterilization were not identified.

[Example 2]

A film having steam permeability was provided to have an area of 13 cm$^2$ for the inlet-side container element. A film having steam permeability was provided to have an area of 16 cm$^2$ for the outlet-side container element. The steam-permeable portion was created for each. Here, a film made of polycarbonate having a thickness of 0.1 mm was used as a film having steam permeability. The thickness D of the filter element at the gripper was configured to 0.95 mm, and the width L of the gripper was configured to 1 mm. The effective filtering area was configured to 46 cm$^2$. The original thickness D0 of the filter element was 9.1 mm. The compressibility ratio by the gripper was 10.4% (compressed to 0.104 times). The blood processing filter was fabricated as with Example 1 except for the aforementioned conditions. The result of an experiment through use of the thus created blood processing filter is shown in Table 1. The leukocyte removing performance was 1.50, which showed high performance. Live bacteria after sterilization were not identified.

[Example 3]

A film having steam permeability was provided to have an area of 13 cm$^2$ for the inlet-side container element. A film having steam permeability was provided to have an area of 16 cm$^2$ for the outlet-side container element. The steam-permeable portion was created for each. A film made of hydrogenated styrene thermoplastic elastomer with a thickness of 0.2 mm was used as a steam-permeable film. The thickness D of the filter element at the gripper was configured to 0.95 mm, and the width L of the gripper was configured to 5 mm. The effective filtering area was configured to 46 cm$^2$. The original thickness D0 of the filter element was 9.1 mm. The compressibility ratio by the gripper was 10.4% (compressed to 0.104 times). The blood processing filter was created as with Example 1 except for the aforementioned conditions. The result of an experiment through use of the thus fabricated blood processing filter is shown in Table 1. The leukocyte removing performance was 1.44, which showed high performance. Live bacteria after sterilization were not identified.

[Example 4]

No film having steam permeability was provided for the inlet-side container element. Only for the outlet-side container element, a film having steam permeability was provided to have an area of 16 cm$^2$, and a steam-permeable portion was created. A film made of soft polyvinyl chloride having a thickness of 0.1 mm was used as a film having steam permeability. The thickness D of the filter element at the gripper was configured to 0.95 mm, and the width L of the gripper was configured to 1 mm. The effective filtering area was configured to 46 cm$^2$. The original thickness D0 of the filter element was 9.1 mm. The compressibility ratio by the gripper was 10.4% (compressed to 0.104 times). The blood processing filter was fabricated as with Example 1 except for the aforementioned conditions. The result of an experiment through use of the thus fabricated blood processing filter is shown in Table 1. The leukocyte removing performance is 1.52, which showed high performance. Live bacteria after sterilization were not identified.

[Comparative Example 1]

The distance at the gripper, that is, the thickness D of the filter element at the gripper was configured to 5 mm, and the width L of the gripper was configured to 5 mm. The original thickness D0 of the filter element was 9.1 mm. The compressibility ratio by the gripper was 54.9% (compressed to 0.549 times). Furthermore, the blood processing filter was fabricated in a manner analogous to that in Example 1 except that no film having steam permeability was disposed. As a result of execution of an examination using this blood processing filter, the leukocyte removing performance was 0.48, which was a lower performance than that in each Example. Live bacteria after sterilization were identified.

[Comparative Example 2]

A film having steam permeability was provided to have an area of 13 cm$^2$ for the inlet-side container element. A film having steam permeability was provided to have an area of 16 cm$^2$ for the outlet-side container element. The steam-permeable portion was created for each. A film made of polycarbonate having a thickness of 0.1 mm was used as a film having steam permeability. The distance at the gripper, that is, the thickness D of the filter element at the gripper was configured to 5.0 mm, and the width L of the gripper was configured to 5.0 mm. The original thickness D0 of the filter element was 9.1 mm. The compressibility ratio by the gripper was 54.9% (compressed to 0.549 times). The blood processing filter was created as with Example 1 except for the aforementioned conditions. As a result of execution of an examination using this blood processing filter, the leukocyte removing performance was 0.52, which was a lower performance than that in each Example. Live bacteria after sterilization were identified.

[Comparative Example 3]

The distance at the gripper, that is, the thickness D of the filter element at the gripper was configured to 0.95 mm, and the width L of the gripper was configured to 5.0 mm. The original thickness D0 of the filter element was 9.1 mm. The compressibility ratio by the gripper was 10.4% (compressed to 0.104 times). Furthermore, the blood processing filter was fabricated in a manner analogous to that in Example 1 except that no film having steam permeability was disposed. As a result of execution of an experiment through use of this blood processing filter, the leukocyte removing performance was 1.52. Meanwhile, live bacteria after sterilization were identified.

[Table 1]

|  | Leukocyte removing capability (-) | Sterilization |
|---|---|---|
| Example 1 | 1.44 | Good |
| Example 2 | 1.5 | Good |
| Example 3 | 1.44 | Good |
| Example 4 | 1.5 | Good |
| Comparative example 1 | 0.48 | Not good |
| Comparative example 2 | 0.52 | Not good |
| Comparative example 3 | 1.52 | Not good |

[Example 5]

The blood processing filter according to Example 5 was fabricated using the method described in the method of manufacturing the blood processing filter 1 according to the aforementioned embodiment. That is, the inlet-side container element was formed by the one mold and the outlet-side container element was formed by the other mold, and subsequently the blood processing filter element was loaded into the outlet-side container element, and the female and male inlet-side container element and outlet-side container element were brought into contact and fitted with each other by moving the mold. Subsequently, the blood processing filter was created using the method of injecting the melt resin through the flow path of the mold formed at the periphery of the fitting portion of the inlet-side container element and the outlet-side container element into the resin flow path of the hard container and of bonding the entire periphery of the fitting portion.
The material of the hard container was polycarbonate resin, the distance of the gripper, that is, the thickness D of the filter element at the gripper was configured to 3.6 mm, the width of the gripper, in other words, the width L of the outer edge portion (the outer edge portion of the filtering surface) of the filter element compressed by the gripper was configured to 5 mm, and the effective filtering area was 46 cm$^2$. A thin-walled portion (having steam permeability) that allows steam to pass was provided to have an area of 13 cm$^2$ for the inlet-side container element. A thin-walled portion having steam permeability was provided to have an area of 16 cm$^2$ for the outlet-side container element. The thickness of each thin-walled portion was configured to be 0.2 mm, and a steam-permeable portion was created for each. Here, the original thickness D0 of the filter element was 9.1 mm. The compressibility ratio by the gripper was 39.6% (compressed to 0.396 times). Polyester nonwoven fabric was stacked according to the following configuration for use as the filter element.

| | |
|---|---|
| Polyester nonwoven fabric 1 (the average fiber diameter was 12 $\mu$m, and the weight per unit area was 30 g/m$^2$) | 6 sheets |
| Polyester nonwoven fabric 2 (the average fiber diameter was 1.6 $\mu$m, and the weight per unit area was 66 g/m$^2$) | 2 sheets |
| Polyester nonwoven fabric 3 (the average fiber diameter was 1.2 $\mu$m, and the weight per unit area was 30 g/m$^2$) | 32 sheets |

The result of an experiment through use of the thus fabricated blood processing filter is shown in Table 2. The leukocyte removing performance is 1.52, which showed high performance. Live bacteria after sterilization were not identified.

[Example 6]

The thickness D of the filter element at the gripper was configured to 0.95 mm, and the width L of the gripper was configured to 1 mm. Here, the original thickness D0 of the filter element was 9.1 mm. The compressibility ratio by the gripper was 10.4% (compressed to 0.104 times). The blood processing filter was fabricated as with Example 1 except for these points. The result of an experiment through use of the thus fabricated blood processing filter is shown in Table 2. The leukocyte removing performance was 1.47, which showed high performance. Live bacteria after sterilization were not identified.

[Example 7]

The thickness D of the filter element at the gripper was configured to 0.95 mm, and the width L of the gripper was configured to 5 mm. The original thickness D0 of the filter element was 9.1 mm. The compressibility ratio by the gripper was 10.4% (compressed to 0.104 times). No thin-walled portion serving as a steam-permeable portion was formed at the inlet-side container element. A thin-walled portion serving as a steam-permeable portion was formed to have an area of 16 cm$^2$ and a thickness of 0.1 mm only at the outlet-side container element. The blood processing filter was created as with Example 1 except for these points. The result of an experiment through use of the thus fabricated blood processing filter is shown in Table 2. The leukocyte removing performance was 1.43, which showed high performance. Live bacteria after sterilization were not identified.

[Example 8]

The thickness D of the filter element at the gripper was configured to 0.95 mm, and the width L of the gripper was configured to 1 mm. The original thickness D0 of the filter element was 9.1 mm. The compressibility ratio by the gripper was 10.4% (compressed to 0.104 times). No thin-walled portion serving as a steam-permeable portion was formed at the inlet-side container element. A thin-walled portion serving as a steam-permeable portion was formed to have an area of 16 cm$^2$ and a thickness of 0.1 mm only at the outlet-side container element. The blood processing filter was fabricated as with Example 1 except for these points. The result of an experiment through use of the thus fabricated blood processing filter is shown in Table 2. The leukocyte removing performance was 1.46, which showed high performance. Live bacteria after sterilization were not identified.

[Comparative Example 5]

The distance at the gripper, that is, the thickness D of the filter element at the gripper was configured to 5 mm, and the width L of the gripper was configured to 5 mm. The original thickness D0 of the filter element was 9.1 mm. The compressibility ratio by the gripper was 54.9% (compressed to 0.549 times). Furthermore, the blood processing filter was fabricated in a manner analogous to that in Example 1 except that no thin-walled portion serving as a steam-permeable portion was formed at the inlet-side container element or the outlet-side container element. As a result of execution of an examination using this blood processing filter, the leukocyte removing performance was 0.4, which was a lower performance than that in each Example. Live bacteria after sterilization were identified.

[Comparative Example 6]

The distance at the gripper, that is, the thickness D of the filter element at the gripper was configured to 3.6 mm, and the width L of the gripper was configured to 0.5 mm. The original thickness D0 of the filter element was 9.1 mm. The compressibility ratio by the gripper was 39.6% (compressed to 0.396 times). Furthermore, the blood processing filter was fabricated in a manner analogous to that in Example 1 except that no film having steam permeability was disposed. As a result of execution of an examination using this blood processing filter, the leukocyte removing performance was 0.44, which was a lower performance than that in each Example. Live bacteria after sterilization were identified.

[Comparative Example 7]

The thickness D of the filter element at the gripper was configured to 5 mm, and the width L of the gripper was configured to 5 mm. The original thickness D0 of the filter element was 9.1 mm. The compressibility ratio by the gripper was 54.9% (compressed to 0.549 times). No thin-walled portion serving as a steam-permeable portion was formed at the inlet-side container element. A thin-walled portion serving as a steam-permeable portion was formed to have an area of 16 cm$^2$ and a thickness of 0.1 mm only at the outlet-side container element. The blood processing filter was created as with Example 1 except for the aforementioned conditions. As a result of execution of an experiment through use of this blood processing filter, the leukocyte removing performance was 0.48. Live bacteria after sterilization were not identified.

[Comparative Example 8]

The distance at the gripper, that is, the thickness D of the filter element at the gripper was configured to 0.95 mm, and the width L of the gripper was configured to 5.0 mm. The original thickness D0 of the filter element was 9.1 mm. The compressibility ratio by the gripper was 10.4% (compressed to 0.104 times). Furthermore, the blood processing filter was fabricated in a manner analogous to that in Example 1 except that no thin-walled portion serving as a steam-permeable portion was formed. As a result of execution of an experiment through use of this blood processing filter, the leukocyte removing performance was 1.52. Meanwhile, live bacteria after sterilization were identified.

[Table 2]

|  | Leukocyte removing capability (-) | Sterilization |
|---|---|---|
| Example 5 | 1.52 | Good |
| Example 6 | 1.47 | Good |
| Example 7 | 1.43 | Good |
| Example 8 | 1.46 | Good |
| Comparative example 5 | 0.4 | Not good |
| Comparative example 6 | 0.44 | Not good |
| Comparative example 7 | 0.48 | Good |
| Comparative example 8 | 1.52 | Not good |

(Method of measuring CV value of thickness of filter element)

An image of a section of the blood processing filter was taken using X-ray CT. The section was selected to pass through the center portion where the thickness of the filter element was the maximum. For example, it was preferable to select the section to pass through the barycenter of the blood processing filter. In the case where ribs provided at the inlet-side container element or the outlet-side container element were linearly arranged, it was preferable to select the section to be perpendicular to the ribs. As to the ribs provided for at least one of the inlet-side container element and the outlet-side container element, at an intermediate point between the ribs and at an intermediate point between the ribs and external periphery, the thicknesses (al, a2, ..., a10) of the filter element were measured at at least three points (preferably at five points, and more preferably at ten points), and the CV value was calculated by dividing the standard deviation by the average value.

(Method of measuring CV value of distance between inlet-side container element and inlet-side surface of filter element)

As to the ribs provided for at least one of the inlet-side container element and the outlet-side container element, at an intermediate point between the ribs and at an intermediate point between the ribs and external periphery, the distance between the inlet-side container element and the inlet-side surface of the filter element were measured at at least three points (preferably at five points), and the CV value was calculated by dividing the standard deviation by the average value.

[Example 9]

The blood processing filter according to Example 9 was fabricated using the method described in the method of manufacturing the blood processing filter 1 according to the aforementioned embodiment. That is, the inlet-side container element was formed by the one mold and the outlet-side container element was formed by the other mold, and subsequently the blood processing filter element was loaded into the outlet-side container element, and the female and male inlet-side container element and outlet-side container element were brought into contact and fitted with each other by moving the mold.

Subsequently, the blood processing filter was fabricated using the method of injecting the melt resin through the flow path of the mold formed at the periphery of the fitting portion of the inlet-side container element and the outlet-side container element into the resin flow path of the hard container and of bonding the entire periphery of the fitting portion. The effective filtering area was configured to have 46 cm$^2$. A thin-walled portion (having steam permeability) that allows steam to pass was provided to have an area of 13 cm$^2$ for the inlet-side container element. A thin-walled portion having steam permeability was provided to have an area of 16 cm$^2$ for the outlet-side container element. The thickness of each thin-walled portion was configured to be 0.2 mm, and a steam-permeable portion was created for each. Polyester nonwoven fabric was stacked according to the following configuration for use as the filter element. The filter element was configured as follows.

| | |
|---|---|
| Polyester nonwoven fabric 1 (the average fiber diameter was 12 $\mu$m, and the weight per unit area was 30 g/m$^2$) | 6 sheets |
| Polyester nonwoven fabric 2 (the average fiber diameter was 1.6 $\mu$m, and the weight per unit area was 66 g/m$^2$) | 2 sheets |
| Polyester nonwoven fabric 3 (the average fiber diameter was 1.2 $\mu$m, and the weight per unit area was 30 g/m$^2$) | 32 sheets |

The material of the hard container was polycarbonate resin, the CV value of the thickness of the filter element was 5.9%, and the CV value of the distance between the inlet side container and the inlet-side surface of the filter element was 9.6%. Through use of the thus fabricated blood processing filter, blood filtering was started, the time until the entire surface on the outlet side being uniformly wet was measured and the result was shown in Table 3.

[Example 10]

| | |
|---|---|
| Polyester nonwoven fabric 1 (the average fiber diameter was 12 $\mu$m, and the weight per unit area was 30 g/m$^2$) | 6 sheets |
| Polyester nonwoven fabric 2 (the average fiber diameter was 1.6 $\mu$m, and the weight per unit area was 66 g/m$^2$) | 2 sheets |
| Polyester nonwoven fabric 3 (the average fiber diameter was 1.2 $\mu$m, and the weight per unit area was 30 g/m$^2$) | 30 sheets |

A polycarbonate resin was adopted as the material of the hard container. The CV value of the thickness of the filter element was 3.6%. The CV value of the distance between the inlet-side container element and the inlet-side surface of the filter element was 10%. A film that allows steam to pass (having steam permeability) was provided to have an area of 13 cm$^2$ for the inlet-side container element. A film having steam permeability was provided to have an area of 16 cm$^2$ for the outlet-side container element. A steam-permeable portion was thus formed for each. The blood processing filter was fabricated in a manner analogous to that in Example 1 except that a film made of polyvinyl chloride having a thickness of 0.2 mm was used as a film having steam permeability.

The result of an experiment through use of this blood processing filter is shown in Table 3.

[Comparative Example 9]

| Polyester nonwoven fabric 1 (the average fiber diameter was 12 μm, and the weight per unit area was 30 g/m²) | 6 sheets |
|---|---|
| Polyester nonwoven fabric 2 (the average fiber diameter was 1.6 μm, and the weight per unit area was 66 g/m²) | 2 sheets |
| Polyester nonwoven fabric 3 (the average fiber diameter was 1.2 μm, and the weight per unit area was 30 g/m²) | 30 sheets |

Table 3 shows a result of measurement of time from start of filtering blood to the point when the entire surface on the outlet side became uniformly wet, through use of a blood processing filter fabricated in a manner analogous to that in Example 1 except that a polycarbonate resin was adopted as the material of the hard container, the CV value of the thickness of the filter element was 3.6%, the CV value of the distance between the inlet-side container element and the inlet-side surface of the filter element was 10%, and furthermore, a film having steam permeability was not provided.

[Comparative Example 10]

The blood processing filter was fabricated by a method of molding the inlet-side container element and the outlet-side container element with molds, subsequently inserting the filter element, and wending the container through ultrasonic bonding. As to the filter, the CV value of the thickness of the filter element was 6.7%. The CV value of the distance between the inlet-side container element and the inlet-side surface of the filter element was 18.6%. A film that allows steam to pass (having steam permeability) was provided to have an area of 13 cm² for the inlet-side container element. A film having steam permeability was provided to have an area of 16 cm² for the outlet-side container element. A steam-permeable portion was formed for each. Table 3 shows a result of measurement of time from start of filtering blood to the point when the entire surface on the outlet side became uniformly wet, through use of a blood processing filter fabricated in a manner analogous to that in Example 1 except that a film made of polyvinyl chloride with a thickness of 0.2 mm was adopted as the film having steam permeability.

[Comparative Example 11]

In Comparative Example 4, a blood processing filter was fabricated using a method of forming the inlet-side container element and the outlet-side container element using the molds, subsequently installing the filter element, and bonding the container by ultrasonic bonding. In Comparative Example 4, the blood processing filter was fabricated as with Example 1 except that the CV value of the thickness of the filter element was 6.7%, the CV value of the distance between the inlet side container element and the inlet-side surface of the filter element was 10%, and no film having steam permeability was provided. Through use of this blood processing filter, blood filtering was started, the time until the entire surface on the outlet side being uniformly wet was measured. The result is shown in Table 3.

[Example 11]

The blood processing filter was fabricated as with Example 1 except that the CV value of the distance between the inlet side container element and the inlet-side surface of the filter element was 18.6%. The result of an experiment through use of this blood processing filter is shown in Table 3.

[Example 12]

The blood processing filter was fabricated as with Example 1 except that the CV value of the thickness of the filter element was 3.6%, and the CV value of the distance between the inlet side container element and the inlet-side surface of the filter element was 18.6%. The result of an experiment through use of this blood processing filter is shown in Table 3.

[Table 3]

|  | CV value (%) of thickness of filter element | CV value (%) of distance between inlet side container element and inlet surface of filter element | Time to entire surface on outlet side being uniformly wet (s) | Sterilization |
|---|---|---|---|---|
| Example 9 | 5.9 | 9.6 | 21 | Good |

(continued)

|  | CV value (%) of thickness of filter element | CV value (%) of distance between inlet side container element and inlet surface of filter element | Time to entire surface on outlet side being uniformly wet (s) | Sterilization |
|---|---|---|---|---|
| Example 10 | 3.6 | 10 | 20 | Good |
| Comparative example 9 | 3.6 | 10 | 21 | Not good |
| Comparative example 10 | 6.7 | 18.6 | 35 | Good |
| Comparative example 11 | 6.7 | 10 | 33 | Not good |
| Example 11 | 5.9 | 18.6 | 21 | Good |
| Example 12 | 3.6 | 18.6 | 22 | Good |

(General result)

As shown in Table 3, in the case of each of the blood processing filters according to Example 9, Example 10, Example 11, and Example 12, the CV value (%) of the thickness of the filter element is 6% or less. In the case of the blood processing filters according to Comparative Examples 10 and 11, the CV value (%) of the thickness of the filter element exceeds 6%. In the case of each of the blood processing filters according to Example 9, Example 10, Example 11, and Example 12, the time for allowing the entire surface on the outlet side to get uniformly wet was 20 to 22 seconds, which was short. On the other hand, in the cases of the blood processing filters according to Comparative Example 10 and Comparative Example 11, the time for causing the entire surface on the outlet side to be uniformly wet was 33 to 35 seconds, which were considerably long. In other words, according to Example 9, Example 10, Example 11 and Example 12, the time in which blood having entered from the inlet was uniformly spread into the inlet space was short. Accordingly, it can be demonstrated that the filter element can be effectively used in comparison with Comparative Examples 10 and 11.

In the case of each of the blood processing filters according to Example 9 and Example 10, the CV value of the distance between the inlet-side container element and the inlet-side surface of the filter element is 10% or less. Meanwhile, in the case of each of the blood processing filters according to Example 11 and Example 12, the CV value of the distance between the inlet-side container element and the inlet-side surface of the filter element exceeds 10%. In comparison between the blood processing filters according to Example 9 and Example 10 and the blood processing filters according to Example 11 and Example 12, there was a tendency that the blood processing filters according to Example 9 and Example 10 require slightly shorter time for making the entire surface on the outlet side be uniformly wet. That is, it can be estimated that the case where the CV value of the distance between the inlet-side container element and the inlet-side surface of the filter element is 10% or less can more effectively use the filter element.

Reference Signs List

1...Blood processing filter, 2...Filter element, 2a, 2b...Filtering surface, 2c...End surface, 2d, 2e...Outer edge portion, 3...Hard container, 3s...Internal space, 4...Inlet-side container element, 4d...Inlet-side fitting portion, 4s...Inlet space, 5...Outlet-side container element, 5d...Outlet-side fitting portion, 5s...0utlet space, 6...Melt resin, 7...Fitting portion, 7a...Sliding contact portion, 9...Gripper, 12...Movable die (one mold), 13...Slidable die (other mold), 31, 31A, 31B...Steam-permeable portion, 31a...Film, 31X...Other portion of hard container, 52b...Close contact portion, L... Width from outermost edge of filter element.

**Claims**

1. A blood processing filter for removing undesirable components from liquid containing a blood component or blood, the filter comprising:

    a sheet-shaped filter element; and

a hard container that includes an inlet-side container element and an outlet-side container element that are disposed to clamp the filter element, and has an internal space separated by the filter element into an inlet space and an outlet space,

wherein the filter element includes a pair of filtering surfaces disposed on the inlet space side and the outlet space side, respectively,

the inlet-side container element and the outlet-side container element are provided with a gripper that clamps and compresses outer edge portions of the pair of filtering surfaces,

the filter element is compressed to have a thickness of a portion clamped by the gripper being 0.05 times or more and 0.5 times or less larger, and

the hard container is provided with a steam-permeable portion having steam permeability.

2. The blood processing filter according to claim 1, wherein the steam-permeable portion is a film that has steam permeability and is provided for at least one of the inlet-side container element and the outlet-side container element.

3. The blood processing filter according to claim 1 or 2, wherein the inlet-side container element and the outlet-side container element are made of a hard resin, and the steam-permeable portion is made of a flexible resin.

4. The blood processing filter according to claim 1, wherein the steam-permeable portion is a part of the hard container and is a thin-walled portion that is thinner than other portions, and the steam-permeable portion and the other portions are integrally formed.

5. The blood processing filter according to claim 4, wherein the steam-permeable portion has a thickness of 50 $\mu$m or more and 500 $\mu$m or less.

6. The blood processing filter according to any one of claims 1 to 5, wherein a coefficient of variation value of a thickness of the filter element is 6% or less.

7. The blood processing filter according to any one of claims 1 to 6, wherein a coefficient of variation value of a distance from the inlet-side container element to an inlet-side surface of the filter element in the inlet space is 10% or less.

8. A blood processing filter for removing undesirable components from liquid containing a blood component or blood, the filter comprising:

a sheet-shaped filter element; and
a hard container that includes an inlet-side container element and an outlet-side container element that are disposed to clamp the filter element, and has an internal space separated by the filter element into an inlet space and an outlet space,

wherein the filter element includes a filtering surface on a side of the inlet space, a filtering surface on a side of the outlet space, and an end surface along peripheries of the pair of filtering surfaces,

the inlet-side container element and the outlet-side container element are provided with a gripper that clamps and compresses outer edge portions of the pair of filtering surfaces,

a coefficient of variation value of a thickness of the filter element is 6% or less, and

the hard container is provided with a steam-permeable portion having steam permeability.

9. The blood processing filter according to claim 8, wherein a coefficient of variation value of a distance from the inlet-side container element to an inlet-side surface of the filter element in the inlet space is 10% or less.

10. The blood processing filter according to claim 8 or 9, wherein the inlet-side container element and the outlet-side container element are made of a hard resin, and the steam-permeable portion is made of a flexible resin.

11. The blood processing filter according to claim 8 or 9, wherein the steam-permeable portion is a part of the hard container and is a thin-walled portion that is thinner than other portions, and the steam-permeable portion and the other portions are integrally formed.

12. The blood processing filter according to claim 11, wherein the steam-permeable portion has a thickness of 50 $\mu$m or more and 500 $\mu$m or less.

13. The blood processing filter according to any one of claims 1 to 12, wherein the filter element includes an end surface along a periphery of the pair of filtering surfaces, and
the gripper includes a close contact portion that is in close contact with the end surface.

14. The blood processing filter according to any one of claims 1 to 13, wherein the gripper compresses the filter element at a range with a width of 1 to 5 mm from an outermost edge of the filter element, as the outer edge portion.

15. The blood processing filter according to claim 13, wherein the inlet-side container element and the outlet-side container element further include a fitting portion that surrounds the end surface of the filter element to be fitted thereto.

16. The blood processing filter according to claim 15, wherein the fitting portion includes an inlet-side fitting portion provided at the inlet-side container element, and an outlet-side fitting portion provided at the outlet-side container element, and at least a part of a sliding contact portion between the inlet-side fitting portion and the outlet-side fitting portion is bonded in a belt shaped manner along an entire periphery of the fitting portion with melt resin.

17. The blood processing filter according to claim 16, wherein the sliding contact portion is provided with a resin flow path, the resin flow path is filled with the melt resin to bond the sliding contact portion.

18. The blood processing filter according to any one of claims 1 to 17, wherein the hard container is made of a resin with a tensile elastic modulus of at least 1 GPa at room temperature.

19. A blood processing filter manufacturing method, the filter being for removing undesirable components from liquid containing a blood component or blood, and comprises a sheet-shaped filter element and a hard container that includes an inlet-side container element and an outlet-side container element that are disposed to clamp the filter element, and has an internal space separated by the filter element into an inlet space and an outlet space, wherein the filter element includes a pair of filtering surfaces disposed on the inlet space side and the outlet space side, the inlet-side container element and the outlet-side container element are provided with a gripper that clamps and compresses outer edge portions of the pair of filtering surfaces, the filter element is compressed by the gripper to have a thickness of being 0.05 to 0.5 times larger, and the hard container is provided with a steam-permeable portion having steam permeability,
the method comprising:

a container element molding step of injection-molding the inlet-side container element with one mold and injection-molding the outlet-side container element with another mold, and forming a steam-permeable portion in at least one of the inlet-side container element and the outlet-side container element;
a inserting step of inserting a filter element into the inlet-side container element or the outlet-side container element;
a joining step of bringing the inlet-side container element and the outlet-side container element into contact with each other in a state where the filter element is loaded, and compressing an outer edge portion of the filter element to have a thickness of being 0.05 to 0.5 times larger; and
a fixing step of fixing the inlet-side container element and the outlet-side container element to each other.

20. The blood processing filter manufacturing method according to claim 19, wherein the container element molding step forms the steam-permeable portion by providing a film having steam permeability for at least one of the inlet-side container element and the outlet-side container element.

21. The blood processing filter manufacturing method according to claim 19, wherein the container element molding step forms the steam-permeable portion by providing a thin-walled portion that is a part thinner than other portions for at least one of the inlet-side container element and the outlet-side container element.

22. The blood processing filter manufacturing method according to any one of claims 19 to 21, wherein the joining step includes a fitting step of mold-fitting the set of molds to fit the inlet-side container element and the outlet-side container element to each other in a state of clamping the filter element.

23. A blood processing filter manufacturing method, the filter being for removing undesirable components from liquid containing a blood component or blood, and comprises a sheet-shaped filter element and a hard container that includes an inlet-side container element and an outlet-side container element that are disposed to clamp the filter element, and has an internal space separated by the filter element into an inlet space and an outlet space, wherein the filter element includes a pair of filtering surfaces disposed on the inlet space side and the outlet space side, the inlet-side container element and the outlet-side container element are provided with a gripper that clamps and compresses outer edge portions of the pair of filtering surfaces, a coefficient of variation value of a thickness of the filter element is 6% or less, and the hard container is provided with a steam-permeable portion having steam permeability,

the method comprising:

a container element molding step of injection-molding the inlet-side container element with one mold and injection-molding the outlet-side container element with another mold, and forming a steam-permeable portion in at least one of the inlet-side container element and the outlet-side container element;

a inserting step of inserting a filter element into the inlet-side container element or the outlet-side container element;

a joining step of bringing the inlet-side container element and the outlet-side container element into contact with each other in a state where the filter element is loaded, and compressing an outer edge portion of the filter element to have a coefficient of variation value being 6% or less; and

a fixing step of fixing the inlet-side container element and the outlet-side container element to each other.

24. The blood processing filter manufacturing method according to claim 23, wherein the container element molding step forms the steam-permeable portion by providing a film having steam permeability for at least one of the inlet-side container element and the outlet-side container element.

25. The blood processing filter manufacturing method according to claim 23, wherein the container element molding step forms the steam-permeable portion by providing a thin-walled portion that is a part thinner than other portions for at least one of the inlet-side container element and the outlet-side container element.

26. The blood processing filter manufacturing method according to any one of claims 23 to 25, wherein the joining step includes a fitting step of mold-fitting the set of molds to fit the inlet-side container element and the outlet-side container element to each other in a state of clamping the filter element

## Patentansprüche

1. Blutverarbeitungsfilter zur Entfernung unerwünschter Komponenten aus einer Flüssigkeit, die eine Blutkomponente enthält, oder aus Blut, wobei der Filter umfasst:

ein blattförmiges Filterelement; und

einen harten Behälter, der ein Behälterelement der Einlassseite und ein Behälterelement der Auslassseite, die so angeordnet sind, dass sie das Filterelement einklemmen, umfasst und einen Innenraum aufweist, der durch das Filterelement in einen Einlassraum und einen Auslassraum aufgeteilt wird;

wobei das Filterelement ein Paar von Filterflächen umfasst, die sich auf der Seite des Einlassraums bzw. auf der Seite des Auslassraums befinden;

das Behälterelement der Einlassseite und das Behälterelement der Auslassseite mit einem Greifer versehen sind, der äußere Randteile des Paars von Filterflächen einklemmt und zusammendrückt;

das Filterelement so zusammengedrückt ist, dass es eine Dicke eines von dem Greifer eingeklemmten Teils aufweist, die das 0,05-fache oder mehr und das 0,5-fache oder weniger beträgt; und

der harte Behälter mit einem dampfdurchlässigen Teil versehen ist, der Dampfdurchlässigkeit aufweist.

2. Blutverarbeitungsfilter gemäß Anspruch 1, wobei der dampfdurchlässige Teil eine Folie ist, die Dampfdurchlässigkeit aufweist und für das Behälterelement der Einlassseite und/oder das Behälterelement der Auslassseite bereitgestellt ist.

3. Blutverarbeitungsfilter gemäß Anspruch 1 oder 2, wobei das Behälterelement der Einlassseite und das Behälterelement der Auslassseite aus einem harten Harz bestehen und der dampfdurchlässige Teil aus einem weichen Harz besteht.

4. Blutverarbeitungsfilter gemäß Anspruch 1, wobei der dampfdurchlässige Teil ein Bestandteil des harten Behälters ist und ein dünnwandiger Teil ist, der dünner als andere Teile ist, und der dampfdurchlässige Teil und die anderen Teile einstückig ausgebildet sind.

**5.** Blutverarbeitungsfilter gemäß Anspruch 4, wobei der dampfdurchlässige Teil eine Dicke von 50 μm oder mehr und 500 μm oder weniger aufweist.

**6.** Blutverarbeitungsfilter gemäß einem der Ansprüche 1 bis 5, wobei der Wert des Variationskoeffizienten der Dicke des Filterelements 6% oder weniger beträgt.

**7.** Blutverarbeitungsfilter gemäß einem der Ansprüche 1 bis 6, wobei der Wert des Variationskoeffizienten des Abstands vom Behälterelement der Einlassseite zur einlassseitigen Oberfläche des Filterelements im Einlassraum 10% oder weniger beträgt.

**8.** Blutverarbeitungsfilter zur Entfernung unerwünschter Komponenten aus einer Flüssigkeit, die eine Blutkomponente enthält, oder aus Blut, wobei der Filter umfasst:

ein blattförmiges Filterelement; und

einen harten Behälter, der ein Behälterelement der Einlassseite und ein Behälterelement der Auslassseite, die so angeordnet sind, dass sie das Filterelement einklemmen, umfasst und einen Innenraum aufweist, der durch das Filterelement in einen Einlassraum und einen Auslassraum aufgeteilt wird;

wobei das Filterelement eine Filterfläche auf einer Seite des Einlassraums, eine Filterfläche auf einer Seite des Auslassraums und eine Endfläche entlang von Rändern des Paars von Filterflächen umfasst;

das Behälterelement der Einlassseite und das Behälterelement der Auslassseite mit einem Greifer versehen sind, der äußere Randteile des Paars von Filterflächen einklemmt und zusammendrückt;

der Wert des Variationskoeffizienten der Dicke des Filterelements 6% oder weniger beträgt; und

der harte Behälter mit einem dampfdurchlässigen Teil versehen ist, der Dampfdurchlässigkeit aufweist.

**9.** Blutverarbeitungsfilter gemäß Anspruch 8, wobei der Wert des Variationskoeffizienten des Abstands vom Behälterelement der Einlassseite zur einlassseitigen Oberfläche des Filterelements im Einlassraum 10% oder weniger beträgt.

**10.** Blutverarbeitungsfilter gemäß Anspruch 8 oder 9, wobei das Behälterelement der Einlassseite und das Behälterelement der Auslassseite aus einem harten Harz bestehen und der dampfdurchlässige Teil aus einem weichen Harz besteht.

**11.** Blutverarbeitungsfilter gemäß Anspruch 8 oder 9, wobei der dampfdurchlässige Teil ein Bestandteil des harten Behälters ist und ein dünnwandiger Teil ist, der dünner als andere Teile ist, und der dampfdurchlässige Teil und die anderen Teile einstückig ausgebildet sind.

**12.** Blutverarbeitungsfilter gemäß Anspruch 11, wobei der dampfdurchlässige Teil eine Dicke von 50 μm oder mehr und 500 μm oder weniger aufweist.

**13.** Blutverarbeitungsfilter gemäß einem der Ansprüche 1 bis 12, wobei das Filterelement eine Endfläche entlang eines Randes des Paars von Filterflächen umfasst; und der Greifer einen Nahkontaktteil umfasst, der sich in engem Kontakt mit der Endfläche befindet.

**14.** Blutverarbeitungsfilter gemäß einem der Ansprüche 1 bis 13, wobei der Greifer das Filterelement in einem Bereich mit einer Breite von 1 bis 5 mm ab einem äußersten Rand des Filterelements als äußerer Randteil zusammendrückt.

**15.** Blutverarbeitungsfilter gemäß Anspruch 13, wobei das Behälterelement der Einlassseite und das Behälterelement der Auslassseite weiterhin ein Befestigungsteil umfassen, das die Endfläche des daran zu befestigenden Filterelements umgibt.

**16.** Blutverarbeitungsfilter gemäß Anspruch 15, wobei das Befestigungsteil ein Befestigungsteil der Einlassseite, das sich am Behälterelement der Einlassseite befindet, und ein Befestigungsteil der Auslassseite, das sich am Behälterelement der Auslassseite befindet, umfasst und wenigstens ein Teil eines Gleitkontaktteils zwischen dem Befestigungsteil der Einlassseite und dem Befestigungsteil der Auslassseite gürtelförmig entlang des ganzen Umfangs des Befestigungsteils mit Schmelzharz verbunden ist.

**17.** Blutverarbeitungsfilter gemäß Anspruch 16, wobei der Gleitkontaktteil mit einem Harzfließweg versehen ist und der Harzfließweg mit dem Schmelzharz gefüllt ist, um den Gleitkontaktteil zu binden.

18. Blutverarbeitungsfilter gemäß einem der Ansprüche 1 bis 17, wobei der harte Behälter aus einem Harz mit einem Zugelastizitätsmodul von wenigstens 1 GPa bei Raumtemperatur besteht.

19. Verfahren zur Herstellung eines Blutverarbeitungsfilters, wobei der Filter zur Entfernung unerwünschter Komponenten aus einer Flüssigkeit, die eine Blutkomponente enthält, oder aus Blut dient und ein blattförmiges Filterelement und einen harten Behälter, der ein Behälterelement der Einlassseite und ein Behälterelement der Auslassseite, die so angeordnet sind, dass sie das Filterelement einklemmen, umfasst und einen Innenraum aufweist, der durch das Filterelement in einen Einlassraum und einen Auslassraum aufgeteilt wird, umfasst, wobei das Filterelement ein Paar von Filterflächen umfasst, die sich auf der Seite des Einlassraums bzw. auf der Seite des Auslassraums befinden, das Behälterelement der Einlassseite und das Behälterelement der Auslassseite mit einem Greifer versehen sind, der äußere Randteile des Paars von Filterflächen einklemmt und zusammendrückt, das Filterelement von dem Greifer so zusammengedrückt ist, dass es eine Dicke aufweist, die das 0,05- bis 0,5-fache beträgt, und der harte Behälter mit einem dampfdurchlässigen Teil versehen ist, der Dampfdurchlässigkeit aufweist;
wobei das Verfahren umfasst:

einen Schritt des Formens der Behälterelemente, bei dem das Behälterelement der Einlassseite mit einer Form spritzgegossen wird und das Behälterelement der Auslassseite mit einer anderen Form spritzgegossen wird und in dem Behälterelement der Einlassseite und/oder dem Behälterelement der Auslassseite ein dampfdurchlässiger Teil gebildet wird;
einen Schritt des Einfügens, bei dem ein Filterelement in das Behälterelement der Einlassseite oder das Behälterelement der Auslassseite eingefügt wird;
einen Schritt des Vereinigens, bei dem das Behälterelement der Einlassseite und das Behälterelement der Auslassseite in einem Zustand, bei dem das Filterelement eingebracht wird, miteinander in Kontakt gebracht werden und ein äußerer Randteil des Filterelements so zusammengedrückt wird, dass er eine Dicke aufweist, die das 0,05-fache bis 0,5-fache beträgt; und
einen Schritt des Fixierens, bei dem das Behälterelement der Einlassseite und das Behälterelement der Auslassseite aneinander fixiert werden.

20. Verfahren zur Herstellung eines Blutverarbeitungsfilters gemäß Anspruch 19, wobei der Schritt des Formens der Behälterelemente den dampfdurchlässigen Teil dadurch bildet, dass er eine Folie, die Dampfdurchlässigkeit aufweist, für das Behälterelement der Einlassseite und/oder das Behälterelement der Auslassseite bereitstellt.

21. Verfahren zur Herstellung eines Blutverarbeitungsfilters gemäß Anspruch 19, wobei der Schritt des Formens der Behälterelemente den dampfdurchlässigen Teil dadurch bildet, dass er einen dünnwandigen Teil, bei dem es sich um einen Bestandteil handelt, der dünner als andere Teile ist, für das Behälterelement der Einlassseite und/oder das Behälterelement der Auslassseite bereitstellt.

22. Verfahren zur Herstellung eines Blutverarbeitungsfilters gemäß einem der Ansprüche 19 bis 21, wobei der Schritt des Vereinigens einen Schritt des Befestigens umfasst, bei dem die Menge der Formen so angepasst wird, dass sie das Behälterelement der Einlassseite und das Behälterelement der Auslassseite in einem Zustand, bei dem das Filterelement eingeklemmt ist, aneinander befestigen.

23. Verfahren zur Herstellung eines Blutverarbeitungsfilters, wobei der Filter zur Entfernung unerwünschter Komponenten aus einer Flüssigkeit, die eine Blutkomponente enthält, oder aus Blut dient und ein blattförmiges Filterelement und einen harten Behälter, der ein Behälterelement der Einlassseite und ein Behälterelement der Auslassseite, die so angeordnet sind, dass sie das Filterelement einklemmen, umfasst und einen Innenraum aufweist, der durch das Filterelement in einen Einlassraum und einen Auslassraum aufgeteilt wird, umfasst, wobei das Filterelement ein Paar von Filterflächen umfasst, die sich auf der Seite des Einlassraums bzw. auf der Seite des Auslassraums befinden, das Behälterelement der Einlassseite und das Behälterelement der Auslassseite mit einem Greifer versehen sind, der äußere Randteile des Paars von Filterflächen einklemmt und zusammendrückt, der Wert des Variationskoeffizienten der Dicke des Filterelements 6% oder weniger beträgt und der harte Behälter mit einem dampfdurchlässigen Teil versehen ist, der Dampfdurchlässigkeit aufweist;
wobei das Verfahren umfasst:

einen Schritt des Formens der Behälterelemente, bei dem das Behälterelement der Einlassseite mit einer Form spritzgegossen wird und das Behälterelement der Auslassseite mit einer anderen Form spritzgegossen wird und in dem Behälterelement der Einlassseite und/oder dem Behälterelement der Auslassseite ein dampfdurchlässiger Teil gebildet wird;

einen Schritt des Einfügens, bei dem ein Filterelement in das Behälterelement der Einlassseite oder das Behälterelement der Auslassseite eingefügt wird;

einen Schritt des Vereinigens, bei dem das Behälterelement der Einlassseite und das Behälterelement der Auslassseite in einem Zustand, bei dem das Filterelement eingebracht wird, miteinander in Kontakt gebracht werden und ein äußerer Randteil des Filterelements so zusammengedrückt wird, dass er einen Wert des Variationskoeffizienten von 6% oder weniger aufweist; und

einen Schritt des Fixierens, bei dem das Behälterelement der Einlassseite und das Behälterelement der Auslassseite aneinander fixiert werden.

24. Verfahren zur Herstellung eines Blutverarbeitungsfilters gemäß Anspruch 23, wobei der Schritt des Formens der Behälterelemente den dampfdurchlässigen Teil dadurch bildet, dass er eine Folie, die Dampfdurchlässigkeit aufweist, für das Behälterelement der Einlassseite und/oder das Behälterelement der Auslassseite bereitstellt.

25. Verfahren zur Herstellung eines Blutverarbeitungsfilters gemäß Anspruch 23, wobei der Schritt des Formens der Behälterelemente den dampfdurchlässigen Teil dadurch bildet, dass er einen dünnwandigen Teil, bei dem es sich um einen Bestandteil handelt, der dünner als andere Teile ist, für das Behälterelement der Einlassseite und/oder das Behälterelement der Auslassseite bereitstellt.

26. Verfahren zur Herstellung eines Blutverarbeitungsfilters gemäß einem der Ansprüche 23 bis 25, wobei der Schritt des Vereinigens einen Schritt des Befestigens umfasst, bei dem die Menge der Formen so angepasst wird, dass sie das Behälterelement der Einlassseite und das Behälterelement der Auslassseite in einem Zustand, bei dem das Filterelement eingeklemmt ist, aneinander befestigen.

## Revendications

1. Filtre de traitement du sang pour éliminer des constituants indésirables d'un liquide contenant un constituant du sang ou du sang, le filtre comprenant :

un élément de filtre en forme de feuille ; et

un récipient dur qui comprend un élément de récipient de côté d'entrée et un élément de récipient de côté de sortie qui sont disposés pour serrer l'élément de filtre, et présente un espace interne séparé par l'élément de filtre dans un espace d'entrée et un espace de sortie,

où l'élément de filtre comprend une paire de surfaces filtrantes disposées sur le côté d'espace d'entrée et le côté d'espace de sortie, respectivement,

l'élément de récipient de côté d'entrée et l'élément de récipient de côté de sortie sont fournis avec une pince qui serre et comprime des portions de bords adjacents de la paire de surfaces filtrantes,

l'élément de filtre est comprimé pour présenter une épaisseur d'une portion serrée par la pince étant 0,05 fois ou plus et 0,5 fois ou moins plus large, et

le récipient dur est muni d'une portion perméable à la vapeur ayant une perméabilité à la vapeur.

2. Filtre de traitement du sang selon la revendication 1, où la portion perméable à la vapeur est un film qui présente une perméabilité à la vapeur et est fourni sur au moins un de l'élément de récipient de côté d'entrée et l'élément de récipient de côté de sortie.

3. Filtre de traitement du sang selon la revendication 1 ou 2, où l'élément de récipient de côté d'entrée et l'élément de récipient de côté de sortie sont constitués d'une résine dure, et la portion perméable à la vapeur est constituée d'une résine flexible.

4. Filtre de traitement du sang selon la revendication 1, où la portion perméable à la vapeur est une partie du récipient dur et est une portion à paroi mince qui est plus mince que d'autres portions, et la portion perméable à la vapeur et les autres portions sont formées de manière intégrale.

5. Filtre de traitement du sang selon la revendication 4, où la portion perméable à la vapeur présente une épaisseur de 50 $\mu$m ou supérieure et de 500 $\mu$m ou inférieure.

6. Filtre de traitement du sang selon l'une quelconque des revendications 1 à 5, où un coefficient de valeur de variation d'une épaisseur de l'élément de filtre est de 6 % ou inférieur.

**7.** Filtre de traitement du sang selon l'une quelconque des revendications 1 à 6, où un coefficient de valeur de variation d'une distance à partir de l'élément de récipient de côté d'entrée jusqu'à une surface de côté d'entrée de l'élément de filtre dans l'espace d'entrée est de 10 % ou inférieur.

**8.** Filtre de traitement du sang pour éliminer des constituants indésirables de liquide contenant un constituant du sang ou du sang, le filtre comprenant :

un élément de filtre en forme de feuille ; et
un récipient dur qui comprend un élément de récipient de côté d'entrée et un élément de récipient de côté de sortie qui sont disposés pour serrer l'élément de filtre, et présente un espace interne séparé par l'élément de filtre dans un espace d'entrée et un espace de sortie,
où l'élément de filtre comprend une surface filtrante sur un côté de l'espace d'entrée, une surface filtrante sur un côté de l'espace de sortie, et une surface d'extrémité le long des périphéries de la paire de surfaces filtrantes,
l'élément de récipient de côté d'entrée et l'élément de récipient de côté de sortie sont munis d'une pince qui serre et comprime des portions de bords externes de la paire de surfaces filtrantes,
un coefficient de valeur de variation d'une épaisseur de l'élément de filtre est de 6 % ou inférieur, et
le récipient dur est muni d'une portion perméable à la vapeur ayant une perméabilité à la vapeur.

**9.** Filtre de traitement du sang selon la revendication 8, où un coefficient de valeur de variation d'une distance à partir de l'élément de récipient de côté d'entrée jusqu'à une surface de côté d'entrée de l'élément de filtre dans l'espace d'entrée est de 10 % ou inférieur.

**10.** Filtre de traitement du sang selon la revendication 8 ou 9, où l'élément de récipient de côté d'entrée et l'élément de récipient de côté de sortie sont constitués d'une résine dure, et la portion perméable à la vapeur est constituée d'une résine flexible.

**11.** Filtre de traitement du sang selon la revendication 8 ou 9, où la portion perméable à la vapeur est une partie du récipient dur et est une portion à paroi mince qui est plus mince que d'autres portions, et la portion perméable à la vapeur et les autres portions sont formées de manière intégrale.

**12.** Filtre de traitement du sang selon la revendication 11, où la portion perméable à la vapeur présente une épaisseur de 50 $\mu$m ou supérieure et de 500 $\mu$m ou inférieure.

**13.** Filtre de traitement du sang selon l'une quelconque des revendications 1 à 12, où l'élément de filtre comprend une surface d'extrémité le long d'une périphérie de la paire de surfaces filtrantes, et
la pince comprend une portion de contact proche qui est en contact proche avec la surface d'extrémité.

**14.** Filtre de traitement du sang selon l'une quelconque des revendications 1 à 13, où la pince comprime l'élément de filtre à un intervalle avec une largeur de 1 à 5 mm à partir d'un bord extérieur de l'élément de filtre, comme la portion de bord externe.

**15.** Filtre de traitement du sang selon la revendication 13, où l'élément de récipient de côté d'entrée et l'élément de récipient de côté de sortie comprennent de plus une portion d'ajustage qui entoure la surface d'extrémité de l'élément de filtre à ajuster à ceux-ci.

**16.** Filtre de traitement du sang selon la revendication 15, où la portion d'ajustage comprend une portion d'ajustage de côté d'entrée fournie sur l'élément de récipient de côté d'entrée, et une portion d'ajustage de côté de sortie fournie sur l'élément de récipient de côté de sortie, et au moins une partie d'une portion de contact de glissement entre la portion d'ajustage de côté d'entrée et la portion d'ajustage de côté de sortie est liée dans une manière en forme de courroie le long d'une périphérie entière de la portion d'ajustage avec de la résine fondue.

**17.** Filtre de traitement du sang selon la revendication 16, où la portion de contact de glissement est munie d'une trajectoire d'écoulement de résine, la trajectoire d'écoulement de résine est remplie avec la résine fondue pour lier la portion de contact de glissement.

**18.** Filtre de traitement du sang selon l'une quelconque des revendications 1 à 17, où le récipient dur est constitué d'une résine avec un module élastique de traction d'au moins 1 GPa à température ambiante.

**19.** Procédé de fabrication de filtre de traitement du sang, le filtre étant destiné à éliminer des constituants indésirables de liquide contenant un constituant du sang ou du sang, et comprend un élément de filtre en forme de feuille et un récipient dur qui comprend un élément de récipient de côté d'entrée et un élément de récipient de côté de sortie qui sont disposés pour serrer l'élément de filtre, et présente un espace interne séparé par l'élément de filtre dans un espace d'entrée et un espace de sortie, où l'élément de filtre comprend une paire de surfaces filtrantes disposées sur le côté d'espace d'entrée et le côté d'espace de sortie, l'élément de récipient de côté d'entrée et l'élément de récipient de côté de sortie sont munis d'une pince qui serre et comprime des portions de bords externes de la paire de surfaces filtrantes, l'élément de filtre est comprimé par la pince pour présenter une épaisseur de 0,05 à 0,5 fois plus large, et le récipient dur est muni d'une portion perméable à la vapeur ayant une perméabilité à la vapeur, le procédé comprenant :

une étape de moulage d'élément de récipient moulant par injection l'élément de récipient de côté d'entrée avec un moule et moulant par injection l'élément de récipient de côté de sortie avec un autre moule, et formant une portion perméable à la vapeur dans au moins un de l'élément de récipient de côté d'entrée et l'élément de récipient de côté de sortie ;
une étape d'insertion insérant un élément de filtre dans l'élément de récipient de côté d'entrée ou l'élément de récipient de côté de sortie ;
une étape de jonction mettant l'élément de récipient de côté d'entrée et l'élément de récipient de côté de sortie en contact l'un avec l'autre dans un état où l'élément de filtre est chargé, et comprimant une portion de bord externe de l'élément de filtre pour qu'elle présente une épaisseur de 0,05 à 0,5 fois plus large ; et
une étape de fixation fixant l'élément de récipient de côté d'entrée et l'élément de récipient de côté de sortie l'un à l'autre.

**20.** Procédé de fabrication de filtre de traitement du sang selon la revendication 19, où l'étape de moulage d'élément de récipient forme la portion perméable à la vapeur en fournissant un film ayant une perméabilité à la vapeur pour au moins un de l'élément de récipient de côté d'entrée et l'élément de récipient de côté de sortie.

**21.** Procédé de fabrication de filtre de traitement du sang selon la revendication 19, où l'étape de moulage d'élément de récipient forme la portion perméable à la vapeur en fournissant une portion à paroi mince qui est une partie plus mince que d'autres portions pour au moins un de l'élément de récipient de côté d'entrée et l'élément de récipient de côté de sortie.

**22.** Procédé de fabrication de filtre de traitement du sang selon l'une quelconque des revendications 19 à 21, où l'étape de jonction comprend une étape d'ajustage ajustant un moule du jeu de moules pour ajuster l'élément de récipient de côté d'entrée et l'élément de récipient de côté de sortie l'un à l'autre dans un état de serrage de l'élément de filtre.

**23.** Procédé de fabrication de filtre de traitement du sang, le filtre étant destiné à éliminer des constituants indésirables de liquide contenant un constituant de sang ou du sang, et comprend un élément de filtre en forme de feuille et un récipient dur qui comprend un élément de récipient de côté d'entrée et un élément de récipient de côté de sortie qui sont disposés pour serrer l'élément de filtre, et présente un espace interne séparé par l'élément de filtre dans un espace d'entrée et un espace de sortie, où l'élément de filtre comprend une paire de surfaces filtrantes disposées sur le côté d'espace d'entrée et le côté d'espace de sortie, l'élément de récipient de côté d'entrée et l'élément de récipient de côté de sortie sont munis d'une pince qui serre et comprime des portions de bords externes de la paire de surfaces filtrantes, un coefficient de valeur de variation d'une épaisseur de l'élément de filtre est de 6 % ou inférieur, et le récipient dur est muni d'une portion perméable à la vapeur ayant une perméabilité à la vapeur, le procédé comprenant :

une étape de moulage d'élément de récipient moulant par injection l'élément de récipient de côté d'entrée avec un moule et moulant par injection l'élément de récipient de côté de sortie avec un autre moule, et formant une portion perméable à la vapeur dans au moins un de l'élément de récipient de côté d'entrée et l'élément de récipient de côté de sortie ;
une étape d'insertion insérant un élément de filtre dans l'élément de récipient de côté d'entrée ou l'élément de récipient de côté de sortie ;
une étape de jonction mettant l'élément de récipient de côté d'entrée et l'élément de récipient de côté de sortie en contact l'un avec l'autre dans un état où l'élément de filtre est chargé, et comprimant une portion de bord externe de l'élément de filtre pour qu'elle présente un coefficient de valeur de variation de 6 % ou inférieur ; et
une étape de fixation fixant l'élément de récipient de côté d'entrée et l'élément de récipient de côté de sortie l'un à l'autre.

**24.** Procédé de fabrication de filtre de traitement du sang selon la revendication 23, où l'étape de moulage d'élément de récipient forme la portion perméable à la vapeur en fournissant un film ayant une perméabilité à la vapeur pour au moins un de l'élément de récipient de côté d'entrée et l'élément de récipient de côté de sortie.

**25.** Procédé de fabrication de filtre de traitement du sang selon la revendication 23, où l'étape de moulage d'élément de récipient forme la portion perméable à la vapeur en fournissant une portion à paroi mince qui est une partie plus mince que d'autres portions pour au moins un de l'élément de récipient de côté d'entrée et l'élément de récipient de côté de sortie.

**26.** Procédé de fabrication de filtre de traitement du sang selon l'une quelconque des revendications 23 à 25, où l'étape de jonction comprend une étape d'ajustage ajustant un moule du jeu de moules pour ajuster l'élément de récipient de côté d'entrée et l'élément de récipient de côté de sortie l'un à l'autre dans un état de serrage de l'élément de filtre.

*Fig.1*

# Fig.2

EP 3 167 918 B1

*Fig.3*

35

*Fig.4*

## Fig.5

# Fig.6

**Fig.7**

## Fig.8

## Fig.9

*Fig.10*

# Fig.11

# *Fig.12*

**EP 3 167 918 B1**

**REFERENCES CITED IN THE DESCRIPTION**

**Patent documents cited in the description**

- JP H11216179 B
- JP H7267871 B
- WO 2004050147 A
- WO 9517236 A
- JP 0526678 A
- JP 2011072814 A